# EUROPEAN PATENT SPECIFICATION

(11) **EP 2 940 127 B1**
(45) Date of publication and mention of the grant of the patent: **25.07.2018**
(21) Application number: 13869167.0
(22) Date of filing: 27.12.2013
(51) Int. Cl.: C12N 5/10

(54) **METHOD FOR PRODUCING INDUCED PLURIPOTENT STEM CELLS, CARDIOMYOCYTES OR PRECURSOR CELLS THEREOF**
VERFAHREN ZUR HERSTELLUNG INDUZIERTER PLURIPOTENTER STAMMZELLEN, KARDIOMYOZYTEN ODER VORLÄUFERZELLEN DAVON
MÉTHODE DE PRODUCTION DE CELLULES SOUCHES PLURIPOTENTES INDUITES, CARDIOMYOCYTES OU CELLULES PRÉCURSEURS CORRESPONDANTES

(30) Priority: 28.12.2012 JP 2012288955
(43) Date of publication of application: 04.11.2015
(73) Proprietor: Kyoto University, Kyoto-shi, Kyoto 606-8501 (JP)
(72) Inventor: KAWAMURA, Teruhisa, Kyoto-shi Kyoto 606-8501 (JP)
(74) Representative: Held, Stephan
(86) International application number: PCT/JP2013/085260
(87) International publication number: WO 2014/104364

(56) References cited:
- WO-A2-03/035838
- JP-A- 2005 224 155
- JP-A- 2005 506 845
- JP-A- 2009 531 021
- YASUYUKI S. KIDA ET AL: "ERRs Mediate a Metabolic Switch Required for Somatic Cell Reprogramming to Pluripotency", CELL STEM CELL, vol. 16, no. 5, 1 May 2015 (2015-05-01), pages 547-555, XP055282856, AMSTERDAM, NL ISSN: 1934-5909, DOI: 10.1016/j.stem.2015.03.001
- HSIN-FU CHEN ET AL: "Surface Marker Epithelial Cell Adhesion Molecule and E-cadherin Facilitate the Identification and Selection of Induced Pluripotent Stem Cells", STEM CELL REVIEWS AND REPORTS, HUMANA PRESS INC, NEW YORK, vol. 7, no. 3, 9 February 2011 (2011-02-09), pages 722-735, XP019927861, ISSN: 1558-6804, DOI: 10.1007/S12015-011-9233-Y
- H.-P. HUANG ET AL: "Epithelial Cell Adhesion Molecule (EpCAM) Complex Proteins Promote Transcription Factor-mediated Pluripotency Reprogramming", JOURNAL OF BIOLOGICAL CHEMISTRY, vol. 286, no. 38, 28 July 2011 (2011-07-28), pages 33520-33532, XP055283094, US ISSN: 0021-9258, DOI: 10.1074/jbc.M111.256164
- BUGANIM YOSEF ET AL: "Single-Cell Expression Analyses during Cellular Reprogramming Reveal an Early Stochastic and a Late Hierarchic Phase", CELL, vol. 150, no. 6, 14 September 2012 (2012-09-14), pages 1209-1222, XP028938733, ISSN: 0092-8674, DOI: 10.1016/J.CELL.2012.08.023
- BRAMBRINK TOBIAS ET AL: "Sequential expression of pluripotency markers during direct reprogramming of mouse somatic cells", CELL STEM CELL, ELSEVIER, CELL PRESS, AMSTERDAM, NL, vol. 2, no. 2, 1 February 2008 (2008-02-01), pages 151-159, XP002484873, ISSN: 1934-5909, DOI: 10.1016/J.STEM.2008.01.004
- JOSE M. POLO ET AL: "A Molecular Roadmap of Reprogramming Somatic Cells into iPS Cells", CELL, vol. 151, no. 7, 1 December 2012 (2012-12-01), pages 1617-1632, XP055282865, US ISSN: 0092-8674, DOI: 10.1016/j.cell.2012.11.039
- WERNIG M ET AL: "In vitro reprogramming of fibroblasts into a pluripotent ES-cell-like state", NATURE, NATURE PUBLISHING GROUP, UNITED KINGDOM, vol. 448, no. 7151, 19 July 2007 (2007-07-19), pages 318-324, XP002621304, ISSN: 0028-0836, DOI: 10.1038/NATURE05944 [retrieved on 2007-06-06]
- IN-HYUN PARK ET AL: "Reprogramming of human somatic cells to pluripotency with defined factors", NATURE, NATURE PUBLISHING GROUP, UNITED KINGDOM, vol. 451, 23 December 2008 (2008-12-23), pages 141-146, XP008153589, ISSN: 0028-0836, DOI: 10.1038/NATURE06534
- ASAKO SHIGENO ET AL.: 'Hyomen Marker ni yoru Shokika Seiko'Fuseiko Gun no Kaiseki' REGENERATIVE MEDICINE vol. 12, February 2013, pages 189 - O-34-7, XP008181707
- TERUHISA KAWAMURA ET AL.: 'Hyomen Marker ni yoru Shokika Seiko Yosoku Inshi no Kaiseki' DAI 33 KAI NIPPON ENSHO'SAISEI IGAKUKAI 05 July 2012, XP008180040
- TERUHISA KAWAMURA ET AL.: 'Shokika Yudo Katei de Shutsugen suru Shinkin Zenkuyo Saibo o Mochiita Anzen Katsu Koritsutekina Shinkin Saisei Ryoho no Kakuritsu' DAI 33 KAI NIPPON ENSHO-SAISEI IGAKUKAI 05 July 2012, XP008180039

## Description

### Technical Field

The present invention relates to a selection method of a somatic cell reprogramming success population. In more detail, the present invention relates to a method of selecting a cell population having high possibility of forming induced pluripotent stem (hereinafter to be referred to as iPS) cells when reprogramming somatic cells. Furthermore, the present invention relates to use of a cell population having low possibility of forming iPS cells, which is produced during the process of reprogramming somatic cells, as a source for the production of myocardial cell.

### Background Art

In recent years, mouse and human iPS cells have been established one after another. Takahashi and Yamanaka (non-patent document 1) induced iPS cells by transferring the Oct3/4, Sox2, Klf4 and c-Myc genes into fibroblasts from a reporter mouse wherein the neomycin resistance gene is knocked-in into the Fbx15 locus, and forcing the cells to express the genes. Okita et al. (non-patent document 2) succeeded in establishing iPS cells (Nanog iPS cells) showing almost the same gene expression and epigenetic profiles as those of embryonic stem (ES) cells, by creating a transgenic mouse having green fluorescence protein (GFP) and puromycin-resistance genes integrated into the locus of Nanog whose expression is confined to pluripotent cells rather than the expression of Fbx15, forcing fibroblasts from the mouse to express the above-mentioned 4 genes, and selecting puromycin-resistant and GFP positive cells. Similar results were also reproduced by other groups (non-patent documents 3, 4). Thereafter, it was revealed that iPS cells could be produced with three of the factors other than c-Myc gene (non-patent document 5).

Furthermore, Takahashi et al. (non-patent document 6) succeeded in establishing iPS cells by transferring into human skin-derived fibroblasts the same four genes as those used in the mouse. On the other hand, Yu et al. (non-patent document 7) produced human iPS cells using Nanog and Lin28 in place of Klf4 and c-Myc. Also, Park et al. (non-patent document 8) produced human iPS cells using TERT and SV40 T antigen, which are known as a human cell immortalizing gene, in addition to the four factors Oct3/4, Sox2, Klf4, c-Myc. Thus, it has been demonstrated that iPS cells comparable to ES cells in differentiation pluripotency can be produced in human and mouse.

However, the efficiency of establishing iPS cells by reprogramming somatic cells has heretofore been reported to be extremely low and several % even in the maximum state. Such low efficiency in establishing iPS cells is one of the maximum factors causing a delay in the elucidation of the molecular mechanism of somatic cell reprogramming, and a solution to this problem is expected to lead to the development of a method of establishing a highly practical, safe, high quality and uniform iPS cell line.

Brambrink et al. (non-patent document 9: "Sequential expression of pluripotency markers during direct reprogramming of mouse somatic cells.") and Buganim Yosef et al. (non-patent document 13: "Single-cell expression analyses during cellular reprogramming reveal an early stochastic and a late hierarchic phase.") analyzed the expression pattern changes of specific genes that occur during reprogramming and identified markers whose expression is linked to successful reprogramming. It has been reported that SSEA1 is expressed in early stages in the process of reprogramming somatic cells to establish iPS cells (non-patent documents 9, 10). In addition, it has been reported that MET (mesenchyme epithelial transition) is an important event in the early stages of somatic cell reprogramming (non-patent documents 11, 12). The use of iPS cells requires not only efficient reprogramming methods, but also their purification. Chen HF et al. used the surface marker EpCAM in order to sort reprogrammed mouse iPS cells (non-patent document 14: "Surface marker epithelial cell adhesion molecule and E-cadherin facilitate the identification and selection of induced pluripotent stem cells.").

Sca-1 (stem cell antigen-1) is an about 18 kDa GPI-anchored surface protein belonging to the Ly-6 family (Ly-6A/E). Sca-1 is expressed in hematopoietic stem cells and hematopoietic progenitor cells in the bone marrow, and used as one of KSL (c-kit+ Sca-1+ Lin-) markers. CD34 is a glycosylated transmembrane protein, and is well-known as a marker of hematopoietic progenitor cells such as hematopoietic stem cells and endothelial stem cells and bone marrow progenitor cells. While the biological function of CD34 has not been analyzed much, it has been shown that CD34 relates to the maintenance of a progenitor cell whose phenotype is in an undifferentiated state. However, it is not known that Sca-1 and CD34 are involved in the success of reprogramming somatic cell.

Using iPS cells, regenerative medicine involving transplantation of somatic cells such as myocardial cells and the like, that have been differentiated ex vivo, into the living body has drastically progressed. However, contamination of the differentiated somatic cells with undifferentiated cells may cause teratoma. Therefore, provision of a cell source with a suppressed risk of teratoma formation is expected in the field of regenerative medicine.

### [Document List]

### [non-patent documents]

non-patent document 1: Takahashi, K. and Yamanaka, S., Cell, 126: 663-676 (2006)
non-patent document 2: Okita, K. et al., Nature, 448: 313-317 (2007)
non-patent document 3: Wernig, M. et al., Nature, 448: 318-324 (2007)
non-patent document 4: Maherali, N. et al., Cell Stem Cell, 1: 55-70 (2007)
non-patent document 5: Nakagawa, M. et al., Nat. Biotethnol., 26: 101-106 (2008)
non-patent document 6: Takahashi, K. et al., Cell, 131: 861-872 (2007)
non-patent document 7: Yu, J. et al., Science, 318: 1917-1920 (2007)
non-patent document 8: Park, I.H. et al., Nature, 451: 141-146 (2008)
non-patent document 9: Cell Stem Cell, 2: 151-159 (2008)
non-patent document 10: Cell Stem Cell, 2: 230-240 (2008)
non-patent document 11: Cell Stem Cell, 7: 64-77 (2010)
non-patent document 12: Cell Stem Cell, 7: 51-63 (2010)
non-patent document 13: Cell, 150: 1209-1222 (2012)
non-patent document 14: Stem Cell Reviews and Reports, 7: 722-735 (2011)

### SUMMARY OF THE INVENTION

### Problems to be Solved by the Invention

The problem of the present invention is to provide a method of selecting a cell population having a high possibility of forming iPS cells in reprogramming somatic cells. Furthermore, the problem of the present invention is to provide a source of myocardial cell or a progenitor cell thereof, which has a low risk of teratoma formation.

### Means of Solving the Problems

The present inventors have conducted intensive studies, took note of two surface markers Sca-1 and CD34, and finally succeeded in concentrating cells that form iPS cells, by sorting Sca-1-negative CD34-negative cell population after transferring reprogramming factors into somatic cells. Furthermore, they have found that while Sca-1-positive CD34-positive cell population shows a low frequency of a cell capable of forming iPS cells, it shows high differentiation potency into myocardial cell and useful as a source of myocardial cell or a progenitor cell thereof with a low risk of teratoma formation. Furthermore, they have comprehensively searched for a gene showing high expression in Sca-1-negative CD34-negative cells by microarray, and found that EpCam is useful as an alternative marker for Sca-1. Based on these findings, they have further studied and completed the present invention. That is, the present invention provides the following:
[1] A method for the production of an induced pluripotent stem cell comprising the following steps:
   (1) contacting nuclear reprogramming substances with somatic cells,
   (2) culturing the cells obtained in (1),
   (3) isolating a Sca-1-negative CD34-negative cell or EpCam-positive CD34-negative cell from the cells obtained in (2), and
   (4) further culturing the cell isolated in (3) to give an induced pluripotent stem cell.
[2] The method of [1], wherein the nuclear reprogramming substances are Oct3/4, Klf4 and Sox2, or nucleic acids encoding the same.
[3] The method of [1] or [2], wherein the nuclear reprogramming substances are Oct3/4, Klf4, Sox2 and c-Myc or L-Myc, or nucleic acids encoding the same.
[4] The method of any of [1] - [3], wherein the somatic cells are rodent somatic cells or human somatic cells.
[5] The method of any of [1] - [4], wherein the somatic cells are fibroblasts.
[6] A method of concentrating a cell capable of forming an induced pluripotent stem cell from somatic cells contacted with nuclear reprogramming substances, comprising isolating a Sca-1-negative CD34-negative cell or EpCam-positive CD34-negative cell from somatic cells contacted with nuclear reprogramming substances.
[7] Use of a reagent comprising an antibody specifically recognizing Sca-1 or EpCam and an antibody specifically recognizing CD34, for concentrating a cell capable of forming an induced pluripotent stem cell from somatic cells contacted with nuclear reprogramming substances.
[8] A method for the production of a myocardial cell or a progenitor cell thereof, comprising the following steps:
   (1') contacting nuclear reprogramming substances with somatic cells,
   (2') culturing the cells obtained in (1'),
   (3') isolating a Sca-1-positive CD34-positive cell or EpCam-negative CD34-positive cell from the cells obtained in (2'), and
   (4') culturing the cell isolated in (3') under a myocardial cell or its progenitor cell differentiation condition to give a myocardial cell or a progenitor cell thereof.
[9] The method of [8], wherein the nuclear reprogramming substances are Oct3/4, Klf4 and Sox2, or nucleic acids encoding the same.
[10] The method of [8] or [9], wherein the nuclear reprogramming substances are Oct3/4, Klf4, Sox2 and c-Myc or L-Myc, or nucleic acids encoding the same.
[11] The method of any of [8] - [10], wherein the somatic cells are rodent somatic cells or human somatic cells.
[12] The method of any of [8] - [11], wherein the somatic cells are fibroblasts.
[13] A method of concentrating a cell which has a low possibility of forming induced pluripotent stem cells but has a high differentiation potency into myocardial cell or a progenitor cell thereof, from somatic cells contacted with nuclear reprogramming substances, comprising isolating a Sca-1-positive CD34-positive cell or EpCam-negative CD34-positive cell from somatic cells contacted with nuclear reprogramming substances.
[14] Use of a reagent comprising an antibody specifically recognizing Sca-1 or EpCam and an antibody specifically recognizing CD34, for concentrating a cell which has a low possibility of forming induced pluripotent stem cells but has a high differentiation potency into myocardial cell or a progenitor cell thereof from somatic cells contacted with nuclear reprogramming substances.

### Effect of the Invention

According to the present invention, since a surface marker important for the prediction of success in reprogramming a somatic cell has been identified, a cell population having high possibility of success in being easily reprogrammed can be isolated in an early stage after transferring reprogramming factors. Moreover, since reprogramming success candidate cells can be narrowed down, more accurate and detailed elucidation of the molecular mechanism relating to the reprogramming is possible. Furthermore, the present invention provides a source of myocardial cell having a low risk of teratoma formation.

### Brief Description of the Drawings

Fig. 1 shows a schematic drawing of a test method of iPS cell production.
Fig. 2 shows expression of Sca-1 and CD34 in the process of somatic cell reprogramming.
Fig. 3 shows expression of Sca-1 and SSEA-1 in the process of somatic cell reprogramming.
Fig. 4 shows expression of SSEA-1, Sca-1 and CD34 in mouse ES cells.
Fig. 5 shows formation of Nanog-positive iPS cell colony from each cell population of Sca-1⁻/CD34⁻, Sca-1⁺/CD34⁺, Sca-1⁺/CD34⁻ sorted on day 2 - 5 after transferring 4 factors.
Fig. 6 shows the analysis results of expression of SSEA-1 by subjecting each cell population of Sca-1⁻/CD34⁻, Sca-1⁺/CD34⁺, Sca-1⁺/CD34⁻ sorted on day 2 - 5 after transferring 4 factors to culture for iPS cell formation.
Fig. 7 shows the comparison results of the number of Nanog-positive iPS cell colonies formed from each cell population of Sca-1⁻/CD34⁻, Sca-1⁺/CD34⁺, Sca-1⁺/CD34⁻ sorted on day 2 - 5 after transferring 4 factors.
Fig. 8 shows the morphology of Sca-1⁻/CD34⁻ cells on day 5 after transferring 4 factors.
Fig. 9 shows the process of iPS cell colony formation from Sca-1⁻/CD34⁻ cells on day 5 after transferring 4 factors.
Fig. 10 shows the process of iPS cell colony formation from Sca-1⁻/CD34⁻ cells on day 5 after transferring 4 factors.
Fig. 11 shows the comparison results of the expression of undifferentiated state marker gene, endoderm marker gene, mesoderm marker gene, ectoderm marker gene in iPS cells derived from Sca-1⁻/CD34⁻ cell population with those in undifferentiated ES cells and differentiated iPS cells. The three columns indicate undifferentiated ES, undifferentiated iPS, differentiated iPS from the left.

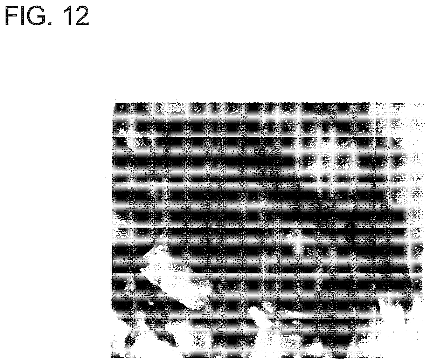

Fig. 12 shows a chimera mouse derived from iPS cells formed from Sca-1⁻/CD34⁻ cells.
Fig. 13 shows the expression of reprogramming factors in each cell population of Sca-1⁻/CD34⁻, Sca-1⁺/CD34⁺, Sca-1⁺/CD34⁻ sorted on day 5 after transferring 4 factors. The three columns indicate Sca-1⁻/CD34⁻, Sca-1⁺/CD34⁺, Sca-1⁺/CD34⁻ from the left.
Fig. 14 shows that the reprogramming efficiency does not change even when the expression level of Oct4 in Sca-1⁻/CD34⁻ cell is increased. The four columns indicate Oct4, Sox2, Klf4, c-Myc from the left.
Fig. 15 shows a schematic drawing of the experimental method of myocardial cell production.
Fig. 16 shows the morphology of the cells obtained by culturing each cell population of Sca-1⁻/CD34⁻, Sca-1⁺/CD34⁺, Sca-1⁺/CD34⁻ in a cardiac progenitor cell induction medium.
Fig. 17 shows spheres of cardiac progenitor cells induced from each cell population of Sca-1⁻/CD34⁻, Sca-1⁺/CD34⁺, Sca-1⁺/CD34⁻.
Fig. 18 shows the comparison results of the number of beating myocardial cell clusters formed from each cell population of Sca-1⁻/CD34⁻, Sca-1⁺/CD34⁺, Sca-1⁺/CD34⁻ sorted on day 5 after transferring 4 factors.
Fig. 19 shows oscillation of the intracellular calcium concentration in beating myocardial cell clusters.
Fig. 20 shows time-course changes in the expression of cardiac progenitor cell marker gene when each cell population of Sca-1⁻/CD34⁻, Sca-1⁺/CD34⁺, Sca-1⁺/CD34⁻ was cultured in a cardiac progenitor cell induction medium.
Fig. 21 shows teratoma formation capacity of each cell of Sca-1⁻/CD34⁻, Sca-1⁺/CD34⁺, Sca-1⁺/CD34⁻.
Fig. 22 shows the comparison results of the number of Nanog-positive iPS cell colonies formed from each cell population of Sca-1⁻/CD34⁻, Sca-1⁺/CD34⁺, Sca-1⁺/CD34⁻ sorted on day 8 after transferring 3 factors.
Fig. 23 shows the results showing that iPS cell was induced at high frequency from Sca-1⁻/CD34⁻ cell population, even when L-Myc was used as a reprogramming factor instead of c-Myc. DN: Sca-1⁻/CD34⁻, DP: Sca-1⁺/CD34⁺, SP: Sca-1⁺/CD34⁻.
Fig. 24 shows the comparison results of the number of human iPS cells induced from each cell population of EpCam⁻ /CD34⁻, EpCam⁺/CD34⁻, EpCam⁻/CD34⁺, EpCam⁺/CD34⁺ sorted on day 7 after transferring 4 factors (Oct3/4, Sox2, Klf4, c-Myc) into human fibroblast.

### Description of Embodiments

The present invention provides a method for the production of an induced pluripotent stem cell comprising the following steps:
(1) contacting nuclear reprogramming substances with somatic cells,
(2) culturing the cells obtained in (1),
(3) isolating a Sca-1-negative CD34-negative cell or EpCam-positive CD34-negative cell from the cells obtained in (2), and
(4) further culturing the cell isolated in (3) to give an induced pluripotent stem cell (production method I of the present invention).

Furthermore, the present invention provides a method for the production of a myocardial cell or a progenitor cell thereof, comprising the following steps:
(1') contacting nuclear reprogramming substances with somatic cells,
(2') culturing the cells obtained in (1'),
(3') isolating a Sca-1-positive CD34-positive cell or EpCam-negative CD34-positive cell from the cells obtained in (2'), and
(4') culturing the cell isolated in (3') under a myocardial cell or its progenitor cell differentiation conditions to give a myocardial cell or a progenitor cell thereof (production method II of the present invention).

In the production methods I and II of the present invention, any cell other than germ cells of mammalian (e.g., mouse, human) origin can be used as a starting material for the production of iPS cell, myocardial cell or a progenitor cell thereof. Examples include keratinizing epithelial cells (e.g., keratinized epidermal cells), mucosal epithelial cells (e.g., epithelial cells of the superficial layer of tongue), exocrine gland epithelial cells (e.g., mammary gland cells), hormone-secreting cells (e.g., adrenomedullary cells), cells for metabolism or storage (e.g., liver cells), intimal epithelial cells constituting interfaces (e.g., type I alveolar cells), intimal epithelial cells of the obturator canal (e.g., vascular endothelial cells), cells having cilia with transporting capability (e.g., airway epithelial cells), cells for extracellular matrix secretion (e.g., fibroblasts), constrictive cells (e.g., smooth muscle cells), cells of the blood and the immune system (e.g., T lymphocytes), sense-related cells (e.g., rod cells), autonomic nervous system neurons (e.g., cholinergic neurons), sustentacular cells of sensory organs and peripheral neurons (e.g., satellite cells), nerve cells and glia cells of the central nervous system (e.g., astroglia cells), pigment cells (e.g., retinal pigment epithelial cells), progenitor cells thereof (tissue progenitor cells) and the like. There is no limitation on the degree of cell differentiation, and the like; even undifferentiated progenitor cells (including somatic stem cells) and finally differentiated mature cells can be used alike as sources of somatic cells in the present invention. Examples of undifferentiated progenitor cells include tissue stem cells (somatic stem cells) such as neural stem cells, hematopoietic stem cells, mesenchymal stem cells, and dental pulp stem cells. The somatic cell is preferably fibroblast.

The choice of mammal to be the source of somatic cells is not particularly limited. Examples thereof include experiment animals such as rodents (e.g., mouse, rat, hamster, guinea pig and the like), rabbit and the like; domestic animals such as swine, bovine, goat, horse, sheep, mink and the like; companion animals such as dog, cat and the like; primates such as human, monkey, Macaca fascicularis, Macaca mulatta, marmoset, orangutan, chimpanzee and the like, and the like. The mammal is preferably a mammal expressing Sca-1, more preferably rodents, most preferably mouse. In a further aspect, the mammal is preferably a mammal expressing EpCam, more preferably rodents or primates, most preferably mouse or human.

When the iPS cell, myocardial cell or progenitor cell thereof obtained by the present invention is used for regenerative medicine in human, the somatic cell is particularly preferably patients' own cell, or cell collected from other person (donor) having the same or substantially the same HLA type as that of the patient, to avoid rejection. The "substantially the same HLA type" as used herein means that the HLA type of the donor is compatible with that of the patient such that transplant cell obtained by inducing differentiation of iPS cell, myocardial cell or progenitor cell thereof derived from a somatic cell of the donor can be transplanted to the patient together with the use of an immunosuppressant and the like. For example, substantially the same HLA type includes an HLA type having the three main HLAs, HLA-A, HLA-B and HLA-DR that matches those of the recipient (the same meaning applies in the following). When the iPS cells, myocardial cells or progenitor cells thereof obtained are not to be administered (transplanted) to a human, but used as, for example, a source of cells for screening for evaluating a patient's drug susceptibility or adverse reactions, it is likewise necessary to collect the somatic cells from the patient or another person with the same genetic polymorphism correlating with the drug susceptibility or adverse reactions.

In the present invention, "a nuclear reprogramming substance" may be configured with any substance, such as a proteinous factor or a nucleic acid that encodes the same (including a form integrated in a vector), or a low molecular compound, as long as it is a substance (substances) capable of inducing an iPS cell from a somatic cell. When the nuclear reprogramming substance is a proteinous factor or a nucleic acid that encodes the same, preferable nuclear reprogramming substance is exemplified by the following combinations (hereinafter, only the names for proteinous factors are shown).
(1) Oct3/4, Klf4, c-Myc
(2) Oct3/4, Klf4, c-Myc, Sox2 (here, Sox2 is replaceable with Sox1, Sox3, Sox15, Sox17 or Sox18; Klf4 is replaceable with Klf1, Klf2 or Klf5; c-Myc is replaceable with T58A (active mutant), N-Myc or L-Myc)
(3) Oct3/4, Klf4, c-Myc, Sox2, Fbx15, Nanog, Eras, ECAT15-2, TclI, β-catenin (active mutant S33Y)
(4) Oct3/4, Klf4, c-Myc, Sox2, TERT, SV40 Large T
(5) Oct3/4, Klf4, c-Myc, Sox2, TERT, HPV16 E6
(6) Oct3/4, Klf4, c-Myc, Sox2, TERT, HPV16 E7
(7) Oct3/4, Klf4, c-Myc, Sox2, TERT, HPV6 E6, HPV16 E7
(8) Oct3/4, Klf4, c-Myc, Sox2, TERT, Bmil
   [For details of these combinations, see WO 2007/069666 (however, in the combination (2) above, for replacement of Sox2 with Sox18, and replacement of Klf4 with Klf1 or Klf5, see Nature Biotechnology, 26, 101-106 (2008)); for details of the combination "Oct3/4, Klf4, c-Myc, Sox2", see also Cell, 126, 663-676 (2006), Cell, 131, 861-872 (2007) and the like. For details of the combination "Oct3/4, Klf4, c-Myc, Sox2, hTERT, SV40 Large T", see also Nature, 451, 141-146 (2008))
(9) Oct3/4, Klf4, Sox2 (see also Nature Biotechnology, 26, 101-106 (2008))
(10) Oct3/4, Sox2, Nanog, Lin28 (see also Science, 318, 1917-1920 (2007))
(11) Oct3/4, Sox2, Nanog, Lin28, hTERT, SV40 Large T (see also Stem Cells Express, published online May 29, 2008, p1-16)
(12) Oct3/4, Klf4, c-Myc, Sox2, Nanog, Lin28 (see also Cell Research (2008) 600-603)
(13) Oct3/4, Klf4, c-Myc, Sox2, SV40 Large T (see also Stem Cells Express, published online May 29, 2008, p1-16)
(14) Oct3/4, Klf4 (see also Nature, Published online, 29 June 2008,p1-5 (doi:10.1038/nature07061))
(15) Oct3/4, c-Myc (see also Nature, Published online, 29 June 2008,p1-5 (doi:10.1038/nature07061))
(16) Oct3/4, Sox2 (see also Nature, 451, 141-146 (2008))

A combination which does not fall in any one of (1) to (16) above, but which comprises all the constituents of any one thereof and an optionally chosen other substance, can also be included in the scope of "nuclear reprogramming substances" in the present invention. Provided that the somatic cell is endogenously expressing one or more of the constituents of any one of (1) to (16) above at a level sufficient to cause nuclear reprogramming, a combination of only the remaining constituents excluding the endogenously expressed constituents can also be included in the scope of "nuclear reprogramming substances" in the present invention.

Among these combinations, when the obtained iPS cell, myocardial cell or progenitor cell thereof is to be used for therapeutic purposes, a combination of 3 factors of Oct3/4, Sox2 and Klf4 (i.e., the above-mentioned (9)) is preferable. On the other hand, when the iPS cell, myocardial cell or progenitor cell thereof is not to be used for therapeutic purposes (e.g., used as an investigational tool for drug discovery screening and the like), 5 factors of Oct3/4, Klf4, c-Myc, Sox2 and Lin28, or 6 factors further including Nanog (i.e., the above-mentioned (12)) is preferable.

Information on the mouse and human cDNA sequences of the aforementioned proteinous factors is available with reference to the NCBI accession numbers mentioned in WO 2007/069666 (In the publication, Nanog is described as ECAT4. Mouse and human cDNA sequence information on Lin28 can be acquired by referring to NCBI accession numbers NM_145833 and NM_024674); those skilled in the art are able to easily isolate these cDNAs. When a proteinous factor is used as it is as a nuclear reprogramming substance, it can be prepared by inserting the cDNA obtained into an appropriate expression vector, transferring it into a host cell, culturing the cell, and recovering the recombinant proteinous factor from the culture. Meanwhile, when a nucleic acid that encodes a proteinous factor is used as a nuclear reprogramming substance, the cDNA obtained is inserted into a viral vector or plasmid vector to construct an expression vector, which is subjected to the nuclear reprogramming step.

Contact of the nuclear reprogramming substance with a somatic cell, when the substance is a proteinaceous factor, can be achieved using a method known per se for protein transfer into a cell. Such methods include, for example, the method using a protein transfer reagent, the method using a protein transfer domain (PTD) fusion protein, the microinjection method and the like. Protein transfer reagents are commercially available, including those based on a cationic lipid, such as BioPOTER Protein Delivery Reagent (Gene Therapy Systems), Pro-Ject™ Protein Transfection Reagent (PIERCE) and ProVectin (IMGENEX); those based on a lipid, such as Profect-1 (Targeting Systems); those based on a membrane-permeable peptide, such as Penetrain Peptide (Q biogene) and Chariot Kit (Active Motif), and the like. The transfer can be achieved per the protocols attached to these reagents, a common procedure being as described below. The nuclear reprogramming substance is diluted in an appropriate solvent (e.g., a buffer solution such as PBS or HEPES), a transfer reagent is added, the mixture is incubated at room temperature for about 5 to 15 minutes to form a complex, this complex is added to cells after exchanging the medium with a serum-free medium, and the cells are incubated at 37°C for one to several hours. Thereafter, the medium is removed and replaced with a serum-containing medium.

Developed PTDs include those using transcellular domains of proteins such as drosophila-derived AntP, HIV-derived TAT, VP22 derived from HSV and the like. A fused protein expression vector incorporating cDNA of a nuclear reprogramming substances and PTD sequence is prepared, and recombination expression is performed using the vector. The fused protein is recovered and used for transfer. Transfer can be performed in the same manner as above except that a protein transfer reagent is not added.

Microinjection, a method of placing a protein solution in a glass needle having a tip diameter of about 1 µm, and injecting the solution into a cell, ensures the transfer of the protein into the cell.

In consideration of the easiness of introduction into a somatic cell, the nuclear reprogramming substance is preferably used in the form of a nucleic acid encoding a proteinaceous factor rather than the proteinaceous factor itself. The nucleic acid may be a DNA or an RNA, or a DNA/RNA chimera. The nucleic acid may be double-stranded or single-stranded. Preferably, the nucleic acid is a double-stranded DNA, particularly cDNA.

cDNA of a nuclear reprogramming substance is inserted into an appropriate expression vector comprising a promoter capable of functioning in a host somatic cell. Useful expression vectors include, for example, viral vectors such as retrovirus, lentivirus, adenovirus, adeno-associated virus, herpes virus, plasmids for the expression in animal cells (e.g., pA1-11, pXT1, pRc/CMV, pRc/RSV, pcDNAI/Neo) and the like. The type of a vector to be used can be chosen as appropriate according to the intended use of the obtained iPS cell, myocardial cell or progenitor cell thereof.

Examples of promoters used in expression vectors include SRα promoter, SV40 promoter, LTR promoter, CMV (cytomegalovirus) promoter, RSV (Rous sarcoma virus) promoter, MoMuLV (Moloney mouse leukemia virus) LTR, HSV-TK (herpes simplex virus thymidine kinase) promoter and the like, with preference given to MoMuLV LTR, CMV promoter, SRα promoter and the like.

The expression vector may contain as desired, in addition to a promoter, an enhancer, a polyadenylation signal, a selectable marker gene, a SV40 replication origin and the like. Examples of selectable marker genes include the dihydrofolate reductase gene, the neomycin resistant gene, and the like.

An expression vector harboring a nucleic acid which is a nuclear reprogramming substance can be introduced into a cell by a technique known per se according to the choice of the vector. In the case of a viral vector, for example, a plasmid containing the nucleic acid is introduced into an appropriate packaging cell (e.g., Plat-E cells) or a complementary cell line (e.g., 293-cells), the viral vector produced in the culture supernatant is recovered, and the vector is infected to a cell by a method suitable for the viral vector. On the other hand, a plasmid vector can be transferred into a cell using the lipofection method, liposome method, electroporation method, calcium phosphate co-precipitation method, DEAE dextran method, microinjection method, gene gun method and the like.

When the nuclear reprogramming substance is a low-molecular-weight compound, the substance can be contacted with a somatic cell by dissolving the substance at a suitable concentration in an aqueous or non-aqueous solvent, adding the solution to a medium suitable for the culture of somatic cell (e.g., minimum essential medium (MEM), Dulbecco's modified Eagle medium (DMEM), RPMI1640 medium, 199 medium, F12 medium and the like containing about 5 - 20% fetal bovine serum. Alternatively, a medium for mesenchymal stem cell such as Mesenchymal stem cell basal medium (Lonza) and the like) such that the concentration of a nuclear reprogramming substance is sufficient to cause nuclear reprogramming in the somatic cell and free of cytotoxicity, and culturing the cells for a given period. While the concentration of the nuclear reprogramming substance varies depending on the kind of the nuclear reprogramming substance to be used, it is appropriately selected from the range of about 0.1 nM - about 100 nM. The contact period may be any as long as it is sufficient for achieving nuclear reprogramming of the cell.

Since the iPS cell establishment efficiency has been low, various substances that improve the efficiency have recently been proposed one after another. It can be expected, therefore, that the iPS cell establishment efficiency will be increased by bringing another establishment efficiency improver, in addition to the aforementioned nuclear reprogramming substance, into contact with the transfer subject somatic cell.

Examples of iPS cell establishment efficiency improvers include, but are not limited to, histone deacetylase (HDAC) inhibitors [e.g., valproic acid (VPA)(Nat. Biotechnol., 26(7): 795-797 (2008)), low-molecular inhibitors such as trichostatin A, sodium butyrate, MC 1293, and M344, nucleic acid-based expression inhibitors such as siRNAs and shRNAs against HDAC (e.g., HDAC1 siRNA Smartpool® (Millipore), HuSH 29mer shRNA Constructs against HDAC1 (OriGene) and the like), and the like], G9a histone methyltransferase inhibitors [e.g., low-molecular inhibitors such as BIX-01294 (Cell Stem Cell, 2: 525-528 (2008)), nucleic acid-based expression inhibitors such as siRNAs and shRNAs against G9a (e.g., G9a siRNA (human) (Santa Cruz Biotechnology) and the like) and the like]. The nucleic acid-based expression inhibitors may be in the form of expression vectors harboring a DNA that encodes an siRNA or shRNA.

Of the aforementioned constituents of nuclear reprogramming substances, SV40 large T, for example, can also be included in the scope of iPS cell establishment efficiency improvers because it is an auxiliary factor unessential for the nuclear reprogramming of somatic cells. While the mechanism of nuclear reprogramming remains unclear, it does not matter whether auxiliary factors, other than the factors essential for nuclear reprogramming, are deemed nuclear reprogramming substances or iPS cell establishment efficiency improvers. Hence, because the somatic cell nuclear reprogramming process is taken as an overall event resulting from contact of a nuclear reprogramming substance and an iPS cell establishment efficiency improver with a somatic cell, it does not always seems to be essential for those skilled in the art to distinguish between the two.

An iPS cell establishment efficiency improver can be contacted with a somatic cell by a method similar to the method mentioned above about the nuclear reprogramming substance for each of (a) when the substance is a proteinous factor and (b) when the substance is a nucleic acid encoding the proteinous factor, or (c) when the substance is a low-molecular-weight compound.

An iPS cell establishment efficiency improver may be contacted with a somatic cell simultaneously with a nuclear reprogramming substance, and either one may be contacted in advance, as far as the iPS cell establishment efficiency from a somatic cell improves significantly compared with the efficiency obtained in the absence of the improver. In an embodiment, for example, when the nuclear reprogramming substance is a nucleic acid that encodes a proteinous factor and the iPS cell establishment efficiency improver is a chemical inhibitor, the iPS cell establishment efficiency improver can be added to the medium after the cell is cultured for a given length of time after the gene transfer treatment, because the nuclear reprogramming substance involves a given length of time lag from the gene transfer treatment to the mass-expression of the proteinous factor, whereas the iPS cell establishment efficiency improver is capable of rapidly acting on the cell. In another embodiment, for example, when the nuclear reprogramming substance and iPS cell establishment efficiency improver are both used in the form of a viral vector or plasmid vector, both may be simultaneously transferred into the cell.

Somatic cell can be pre-cultured using a medium known per se suitable for their cultivation. For example, it can be pre-cultured in minimal essential medium (MEM) containing about 5 to 20% fetal bovine serum, Dulbecco's modified Eagle medium (DMEM), RPMI1640 medium, 199 medium, F12 medium, and the like.

When a transfer reagent such as cationic liposome and the like is used in contacting with nuclear reprogramming substances (and an iPS cell establishment efficiency improver), it is sometimes preferable that the medium have been replaced with a serum-free medium so as to prevent the transfer efficiency from decreasing. After being contacted with nuclear reprogramming substances (and iPS cell establishment efficiency improver), the somatic cell is cultured. The culture is performed under, for example, conditions suitable for cultivation of ES cells. In the case of human cells, it is preferable to add a basic fibroblast growth factor (bFGF) as an inhibitor of differentiation to a conventional medium and perform cultivation. In the case of mouse cells, it is desirable to add a Leukemia Inhibitory Factor (LIF) instead of bFGF. Usually, the cell is cultured in the co-presence of mouse embryonic fibroblasts (MEFs) treated with radiation or an antibiotic to terminate the cell division, as feeder cells. Usually, STO cells and the like are commonly used as MEFs; for induction of an iPS cell, however, the SNL cell [McMahon, A.P. & Bradley, A. Cell 62, 1073-1085 (1990)] and the like are commonly used.

The time of cultivation of somatic cells contacted with nuclear reprogramming substances is not particularly limited as long as it is sufficient to induce expression of Sca-1 and/or CD34 in a partial cell population of the somatic cells as a result of the contact with the nuclear reprogramming substances, and distinguish Sca-1-negative CD34-negative cell population from Sca-1-positive CD34-positive cell population by flow cytometry analysis. In a further aspect, the time of cultivation of somatic cells contacted with nuclear reprogramming substances is not particularly limited as long as it is sufficient to induce expression of EpCam and/or CD34 in a partial cell population of the somatic cell as a result of the contact with the nuclear reprogramming substances, and distinguish EpCam-positive CD34-negative cell population from EpCam-negative CD34-positive cell population by flow cytometry analysis. Generally, Sca-1-negative CD34-negative cell population is sufficiently distinguished from Sca-1-positive CD34-positive cell population, or EpCam-positive CD34-negative cell population is sufficiently distinguished from EpCam-negative-CD34 positive cell population by flow cytometry analysis 2 days after contact with the nuclear reprogramming substances. Therefore, the time of cultivation of somatic cells contacted with nuclear reprogramming substances is generally not less than 2 days, preferably not less than 3 days, more preferably not less than 4 days. When the culture period is too long, iPS cell with a higher proliferation rate emerges to overwhelm non-iPS cells, and the object of isolation of a fraction wherein non-iPS cells in a reprogramming process are concentrated, which forms iPS cell, cannot be achieved. Therefore, the culture period is generally within 13 days, preferably within 9 days, more preferably within 5 days. Therefore, the time of cultivation of somatic cells contacted with nuclear reprogramming substances is generally 2 - 13 days, preferably 3 - 9 days, more preferably 4 - 5 days. When a somatic cell of primates such as human and the like is used, induction of iPS cell takes a longer time as compared to rodents. Thus, the time of cultivation of somatic cells contacted with nuclear reprogramming substances is generally 2 - 20 days, preferably 4 - 15 days, more preferably 6 - 7 days.

When production of iPS cell is finally intended, a Sca-1-negative CD34-negative cell or EpCam-positive CD34-negative cell is isolated from the cells after the above-mentioned culture.

When production of myocardial cell or a progenitor cell thereof is finally intended, a Sca-1-positive CD34-positive cell or EpCam-negative CD34-positive cell is isolated from the cells after the above-mentioned culture.

Sca-1 (stem cell antigen-1) is a known GPI-anchored surface protein of about 18 kDa belonging to the Ly-6 family (Ly-6A/E). In mouse, two gene products of Ly6-A/E allyl, i.e., Ly-6E.1 and Ly-6A.2, are expressed in different mouse strain, both being encompassed in Sca-1. The information of a representative amino acid sequence of mouse Sca-1 can be obtained by making reference to NCBI accession number NP_034868.1 (updated on December 12, 2012). The information of a representative amino acid sequence of Rat Sca-1 can be obtained by making reference to NCBI accession number XP_216959.1 (updated on June 20, 2012).

CD34 is a glycosylated transmembrane protein and is well known as a marker of hematopoietic progenitor cells and bone marrow progenitor cell, such as hematopoietic stem cell and endothelial stem cell. The information of a representative amino acid sequence of mouse CD34 can be obtained by making reference to NCBI accession number NP_598415.1 (updated on December 12, 2012). The information of a representative amino acid sequence of rat CD34 can be obtained by making reference to NCBI accession number NP_001100672.1 (updated on August 27, 2012). The information of a representative amino acid sequence of human CD34 can be obtained by making reference to NCBI accession number NP_001020280.1 (updated on December 9, 2012).

EpCam (Epithelial cell adhesion molecule) is a transmembrane glycoprotein that mediates cell-cell adhesion in epithelium independently from calcium ion. The information of a representative amino acid sequence of mouse EpCam can be obtained by making reference to NCBI accession number NP_032558.2 (updated on October 27, 2013). The information of a representative amino acid sequence of rat EpCam can be obtained by making reference to NCBI accession number NP_612550.1 (updated on September 2, 2013). The information of a representative amino acid sequence of human EpCam can be obtained by making reference to NCBI accession number NP_002345.2 (updated on December 21, 2013).

In the present specification, when cell phenotype is indicated by positivity/negativity of marker molecule (antigen) expression, unless otherwise specified, the cell phenotype is indicated by the presence or absence of a specific bond due to an antibody specifically recognizing the marker molecule. The cell phenotype by positivity/negativity of marker molecule expression is determined by flow cytometry analysis using a specific antibody to the marker molecule and the like. The marker molecule being "positive" means that the marker molecule is expressed on the cellular surface and a specific bond by an antibody specifically recognizing the marker molecule can be confirmed. The marker molecule being "negative" means that the marker molecule is not expressed on the cellular surface and a specific bond by an antibody specifically recognizing the marker molecule cannot be confirmed.

Sca-1-negative CD34-negative cell or Sca-1-positive CD34-positive cell can be isolated by staining the cells after the above-mentioned culture with an antibody specifically recognizing Sca-1 and an antibody specifically recognizing CD34, and sorting the object fractions (i.e., Sca-1-negative CD34-negative fraction or Sca-1-positive CD34-positive fraction) by using a cell sorter, a magnetic column and the like. To achieve high purity, a cell sorter is preferably used.

EpCam-positive CD34-negative cell or EpCam-negative CD34-positive cell can be isolated by staining the cells after the above-mentioned culture with an antibody specifically recognizing EpCam and an antibody specifically recognizing CD34, and sorting the object fractions (i.e., EpCam-positive CD34-negative fraction or EpCam-negative CD34-positive fraction) by using a cell sorter, a magnetic column and the like. To achieve high purity, a cell sorter is preferably used.

In the present specification, "isolation" means that an operation to remove factors (or cells) other than the object components (or cells) has been done, and the state of natural presence has already passed. The purity of the "isolated Sca-1-negative CD34-negative cell" (percentage of the number of Sca-1-negative CD34-negative cells in the total number of cells) is generally not less than 70%, preferably not less than 80%, more preferably not less than 90%, most preferably 100%. This also applies to the "Sca-1-positive CD34-positive cell" and other cells.

When production of iPS cell is finally intended, the isolated Sca-1-negative CD34-negative cell or EpCam-positive CD34-negative cell is further cultured under the same culture conditions as those of somatic cell after contact with the aforementioned nuclear reprogramming substances (and iPS cell establishment efficiency improving substance), whereby iPS cell is obtained.

A candidate colony of iPS cells can be selected in two ways: methods with drug resistance and reporter activity as indicators, and methods based on macroscopic examination of morphology. As an example of the former, a colony positive for drug resistance and/or reporter activity is selected using a recombinant cell wherein the locus of a gene highly expressed specifically in pluripotent cells (e.g., Fbx15, Nanog, Oct3/4 and the like, preferably Nanog or Oct3/4) is targeted by a drug resistance gene and/or a reporter gene. On the other hand, methods for selecting a candidate colony by macroscopic examination of morphology include, for example, the method described by Takahashi et al. in Cell, 131, 861-872 (2007). Although the methods using reporter cells are convenient and efficient, colony selection by macroscopic examination is desirable from the viewpoint of safety when iPS cells are prepared for therapeutic purposes in humans. When 3 factors of Oct3/4, Klf4 and Sox2 are used as the nuclear reprogramming substance, the number of the established clones decreases, but almost all resulting colonies are iPS cells having high quality comparable to that of ES cell. Therefore, iPS cell can be established efficiently even without using a reporter cell.

Whether the cell of the selected colony is an iPS cell can be confirmed by various test methods known per se, for example, expression analysis of ES cell-specific gene (undifferentiated marker gene) and the like described in the Examples below. When more accuracy is desired, the selected cell may be transplanted into a mouse and teratoma formation is confirmed. Alternatively, the differentiation potency into all germ layers including germ lines (pluripotency) can be confirmed by introducing the selected cells into a host embryo to prepare a chimera embryo, transferring the chimera embryo into a pseudopregnant animal to give a chimera animal, further crossing the chimera animal with a normal animal or the same kind of chimera animal, and confirming that an offspring animal of the selected cell is contained in the next generation (F1) animals.

The iPS cells thus established can be used for various purposes. For example, by utilizing a method of differentiation induction reported with respect to ES cells, differentiation into various cells (e.g., myocardial cell, retina cells, blood cells, nerve cells, vascular endothelial cells, insulin-secreting cells and the like), tissue and organ from iPS cells can be induced.

When production of myocardial cell or a progenitor cell thereof is finally intended, the isolated Sca-1-positive CD34-positive cell or EpCam-negative CD34-positive cell is further cultured under the conditions for differentiation into myocardial cell or a progenitor cell thereof and myocardial cell or a progenitor cell thereof is obtained.

As the differentiation conditions into myocardial cell or a progenitor cell thereof, any method known as a method of inducing differentiation of ES cell or iPS cell into myocardial cell or a progenitor cell thereof can be adopted.

For example, cardiac progenitor cell can be induced by adhesion culture of cells in a serum-free medium containing bFGF and EGF (Tomita Y et al. J Cell Biol. 2005; 170:1135-46). Suspension culture of cardiac progenitor cell forms a sphere, and adhesion culture of the sphere in a serum-containing medium develops myocardial cell in the sphere (Tomita Y et al. J Cell Biol. 2005; 170:1135-46). Alternatively, differentiation into myocardial cell can be induced by culturing cells in the presence of SCF and on OP9 stroma feeder (Proc. Natl. Acad. Sci. USA, vol.100, p4018-4023, 2003). Differentiation into myocardial cell can be induced by culturing cells on a type IV collagen-coated culture container in a medium free of LIF induces Flk+ cells, sorting the Flk+ cells, and culturing the cells on OP9 stroma feeder (Yamashita JK et al. FASEB J. 2005; 19:1534-1536, Narazaki G et al. Circulation. 2008; 118:498-506).

Differentiation of the obtained cells into cardiac progenitor cells can be confirmed by evaluating expression and the like of a cardiac progenitor cell specific gene.

Differentiation of the obtained cells into myocardial cell can be confirmed by evaluating self-beating of cell and sphere, oscillation of intracellular calcium concentration that changes synchronized with self-beating, expression of myocardial cell specific gene and the like.

By transplanting the finally-obtained myocardial cell and progenitor cell thereof into the cardiac muscle, various cardiac muscle diseases (cardiomyopathy, myocarditis, heart tumor etc.) caused by disorders of cardiac muscle can be treated.

The present invention also provides the use of a reagent (kit) containing an antibody specifically recognizing Sca-1 and an antibody specifically recognizing CD34, for concentrating a cell capable of forming an induced pluripotent stem cell from somatic cells contacted with nuclear reprogramming substances (reagent I of the present invention). Using reagent I of the present invention, an induced pluripotent stem cell can be produced with ease by isolating Sca-1-negative CD34-negative cell in the above-mentioned production method I of the present invention.

In a further aspect, the present invention provides the use of a reagent (kit) containing an antibody specifically recognizing EpCam and an antibody specifically recognizing CD34, for concentrating a cell capable of forming an induced pluripotent stem cell from somatic cells contacted with nuclear reprogramming substances (reagent I' of the present invention). Using reagent I' of the present invention, an induced pluripotent stem cell can be produced with ease by isolating EpCam-positive CD34-negative cell in the above-mentioned production method I of the present invention.

Furthermore, the present invention provides the use of a reagent (kit) containing an antibody specifically recognizing Sca-1 and an antibody specifically recognizing CD34, for concentrating a cell which has a low possibility of forming induced pluripotent stem cells but has a high differentiation potency into myocardial cell or a progenitor cell thereof, from somatic cells contacted with nuclear reprogramming substances (reagent II of the present invention).
Using reagent II of the present invention, myocardial cell or a progenitor cell thereof can be produced with ease by isolating Sca-1-positive CD34-positive cell in the above-mentioned production method II of the present invention.

In a further aspect, the present invention provides the use of a reagent (kit) containing an antibody specifically recognizing EpCam and an antibody specifically recognizing CD34 for concentrating a cell which has a low possibility of forming induced pluripotent stem cells but has a high differentiation potency into myocardial cell or a progenitor cell thereof, from somatic cells contacted with nuclear reprogramming substances (reagent II' of the present invention). Using reagent II' of the present invention, myocardial cell or a progenitor cell thereof can be produced with ease by isolating EpCam-negative CD34-positive cell in the above-mentioned production method II of the present invention.

In the present specification, "specific recognition" of antigen X by an antibody means that the binding affinity of an antibody for antigen X in an antigen-antibody reaction is higher than that of non-specific antigen (e.g., BSA).

In the present specification, examples of the antibody include, but are not limited to, natural antibodies such as polyclonal antibody, monoclonal antibody (mAb) and the like; and recombinant antibodies produced using a gene recombinant technique (e.g., single-chain Fv fragments (scFv), bispecific-chimeric scFV (χ-scFv), tandem scFV (scFv)₂, bispecific-(scFv)₂, disulfide-linked scFv, disulfide-stabilized Fv fragment (dsFv), diabody, single-chain diabody (scDb), bivalent diabody, bispecific diabody, knob-into-hole stabilized diabody, disulfide-stabilized diabody, triabody, tetrabody, trispecific triabody, CL-dimerized scFv, CH1-CL-dimerized scFv, CH3-dimerized scFv, knob-into-hole CH3-dimerized scFv, CH3-dimerized bivalent diabody, Fc-dimerized scFv, Fab-scFv fusions, Ig-scFv fusions, leucine-zipper stabilized scFv dimers, helix-stabilized scFv dimers, 4 helix-bundle stabilized scFv tetramers, streptavidin-scFv, intrabody, Fab' fragments, F(ab') fragments, Fv fragments (Fv), chimera antibody, humanized antibody, single strand antibody etc.), human antibodies that can be produced using a human antibody producing transgenic animal and the like, and the like. As the antibody, a polyclonal antibody or a monoclonal antibody is preferably used in view of easy preparation and the like. Also, the class of antibody is not particularly limited, and any antibody having any isotype from IgG, IgM, IgA, IgD and IgE and the like is also encompassed.

In the present invention, moreover, "antibody" is a concept also including a binding fragment thereof. The binding fragment of an antibody means a partial region of the aforementioned antibody, and specific examples thereof include F(ab')₂, Fab', Fab, Fv (variable fragment of antibody), scFv, dsFv (disulfide stabilized Fv), dAb (single domain antibody) and the like (Exp. Opin. Ther. Patents, Vol.6, No.5, p.441-456, 1996), antibody fragment prepared by Fab expression library and the like.

An antibody specifically recognizing Sca-1 and an antibody specifically recognizing CD34 can be produced by respectively immunizing a non-human mammal with polypeptide of Sca-1 and CD34, or a partial peptide having antigenicity thereof, and isolating an antibody specifically recognizing Sca-1, and an antibody specifically recognizing CD34, respectively.

An antibody specifically recognizing EpCam can be produced by immunizing a non-human mammal with polypeptide of EpCam, or a partial peptide having antigenicity thereof, and isolating an antibody specifically recognizing EpCam.

When a monoclonal antibody is produced, an antigen polypeptide is administered alone or together with carrier, diluent etc. to a non-human mammal (mouse, rat, hamster etc.) to immunize the non-human mammal with the phosphorylation polypeptide or conjugate, antibody producing cells contained in the spleen and lymph node are fused with myeloma cells according to, for example, the method of Köhler and Milstein [Nature, 256, 495 (1975)] and a known variant method thereof to establish a monoclonal antibody producing hybridoma, a hybridoma producing a monoclonal antibody specifically recognizing the object antigen is selected by ELISA and the like, and a monoclonal antibody specifically recognize the object antigen (Sca-1/CD34) is isolated from the culture supernatant of the above-mentioned hybridoma or ascites obtained by transferring the above-mentioned hybridoma into a pristine-treated nude mouse.

When a polyclonal antibody is produced, an antigen polypeptide is administered alone or together with carrier, diluent etc. to a non-human mammal (rabbit, sheep, goat etc.) to immunize the mammal with the conjugate, and an antibody fraction is isolated from serum, ascites and the like, preferably serum, of the immunized non-human mammal, whereby a polyclonal antibody specifically recognizing the object antigen (Sca-1/CD34) can be obtained.

An antibody specifically recognizing Sca-1, an antibody specifically recognizing CD34, and an antibody specifically recognizing EpCam may be each labeled with a fluorescence dye (FITC, PE etc.), biotin, enzyme (alkaliphosphatase, peroxidase), magnetic bead and the like.

The reagents (kits) I and II used according to the present invention may contain various reagents used for practicing the aforementioned production method of the present invention, in addition to an antibody specifically recognizing Sca-1 and an antibody specifically recognizing CD34. Examples of the reagent include the aforementioned nuclear reprogramming substances, medium, magnetic bead and the like. When a human iPS cell is produced, the reagent I used according to the present invention can further contain bFGF. When a mouse iPS cell is produced, the reagent I used according to the present invention can further contain LIF. The reagent II used according to the present invention can further contain cytokine (bFGF, EGF, SCF etc.), OP9 stromal cell and the like useful for the differentiation induction into myocardial cell and cardiac progenitor cell.

The reagents (kits) I' and II' used according to the present invention may contain various reagents used for practicing the aforementioned production method of the present invention, in addition to an antibody specifically recognizing EpCam and an antibody specifically recognizing CD34. Examples of the reagent include the aforementioned nuclear reprogramming substances, medium, magnetic bead and the like. When a human iPS cell is produced, the reagent I' used according to the present invention can further contain bFGF. When a mouse iPS cell is produced, the reagent I' used according to the present invention can further contain LIF. The reagent II' used according to the present invention can further contain cytokine (bFGF, EGF, SCF etc.), OP9 stromal cell and the like useful for the differentiation induction into myocardial cell and cardiac progenitor cell.

### Examples

The present invention is explained in more detail in the following by referring to Examples.

### [Example 1] Production of induced pluripotent stem cells

According to the method described in Takahashi, K. and Yamanaka, S., Cell, 126: 663-676 (2006), 4 factors (Oct3/4, Sox2, Klf4, c-Myc) derived from mouse were transferred into mouse embryonic fibroblasts by a retrovirus, and the fibroblasts were cultured in a medium (FBS mES medium without LIF). The medium composition is as described below.

**Table 1**

| |
|---|
| DMEM (Invitrogen, 11995) 425ml |
| Fetal Bovine Serum (ES grade, heat inactivated) 50ml |
| 100x Non-essential amino acid (Millipore, TMS-001-C) 5ml |
| 100x Nucleosides (Millipore, ES-008-D) 5ml |
| Sodium pyruvate (x100) (Invitrogen, 11360-070) 5ml |
| Penicillin-Streptmycin(x100) (Invitrogen, 15140-122) 5ml |
| Glutamax(x100) (Invitrogen, 35050-061) 5ml |
| 2-Mercaptoethanol (x1000) (Invitrogen, 21985-023) 1ml |
| **(LIF (Millipore) was added at 1000U/ml final immediately before use)** |

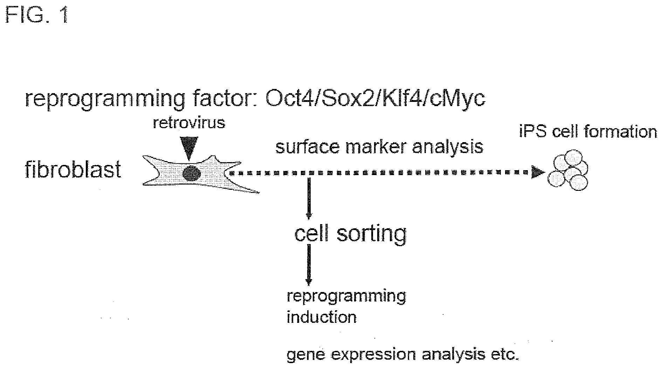

Over time, the expression of Sca-1, CD34 and SSEA1 was analyzed by flow cytometry, desired cell population was isolated by cell sorter, and replated on feeder cells. As the feeder cell, MEF treated with mitomycin C to stop cell division was used (Fig. 1).

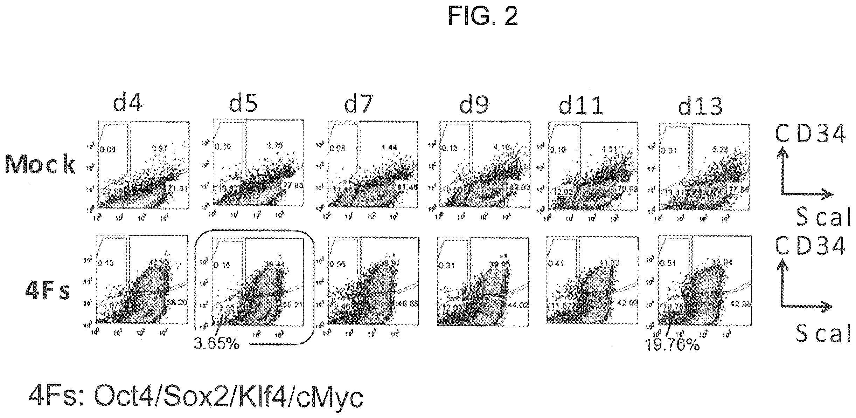

The time-course changes in Sca-1 and CD34 expression are shown in Fig. 2. Of the two markers, of Sca-1 is originally expressed in about 70% of mouse fibroblasts (MEFs), and the positive rate increased to about 95% with a peak appearing on day 5 by the introduction of the 4 factors (Oct3/4, Sox2, Klf4, c-Myc). Expression of CD34 was not observed in the control (Mock introduction), but about 30 - 40% of positive cells emerged from days 4 - 5, by the introduction of the 4 factors (Oct3/4, Sox2, Klf4, c-Myc).

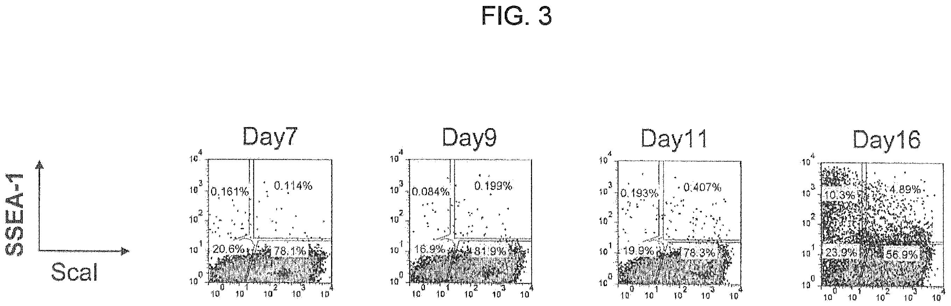

On the other hand, cells positive for SSEA1 scarcely appeared from the introduction of the 4 factors (Oct3/4, Sox2, Klf4, c-Myc) up to day 11, and positive cells emerged from day 16 (Fig. 3).

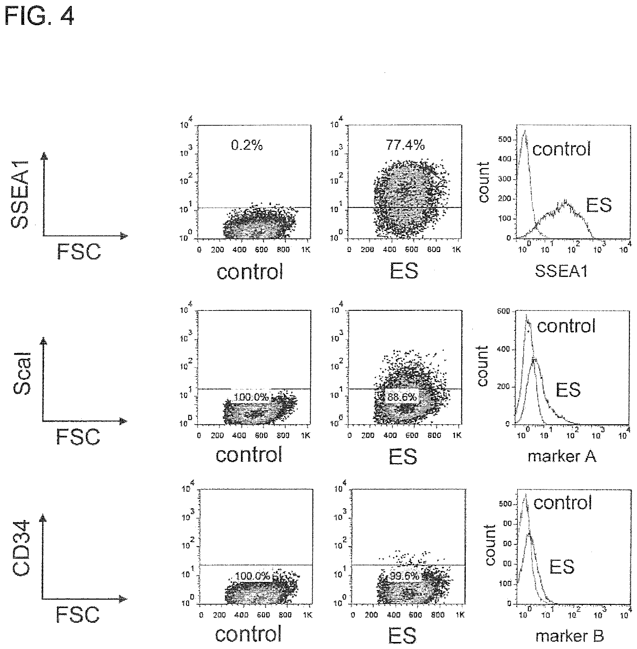

For reference, expression of Sca-1, CD34 and SSEA1 in mouse ES cells was analyzed by flow cytometry (Fig. 4). Mouse ES cell was SSEA1-positive. As for Sca-1 and CD34, a slight peak shift was observed.

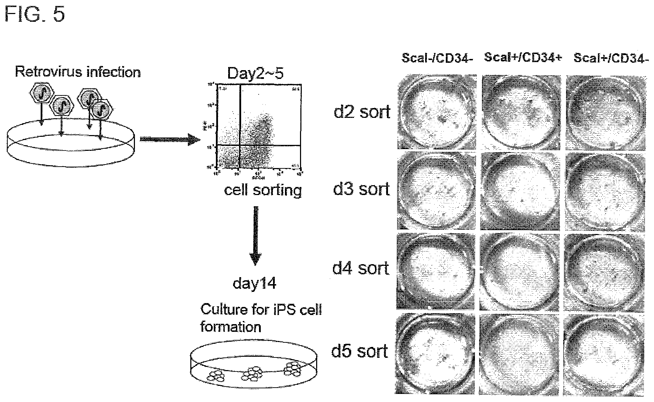

On day 2 - 5 from introduction of the 4 factors, each cell population of Sca-1⁻/CD34⁻, Sca-1⁺/CD34⁺, Sca-1⁺/CD34⁻ was sorted by a cell sorter, the cells were replated on feeder cells and cultured again under conditions suitable for iPS cell formation. On day 14 of culture, the colonies of iPS cells were visualized by an anti-Nanog antibody (Fig. 5). When sorted on day 2 from the introduction of the 4 factors, the frequency of emergence of iPS cell colonies does not vary among 3 fractions, but when sorted on day 3, the frequency of emergence of iPS cell colonies in Sca-1⁻/CD34⁻ cell population tended to be high. When sorted on day 4 and day 5 or after, iPS cells mainly emerged from Sca-1⁻/CD34⁻ cell population. Therefore, a possibility of strong fate determination to be iPS cell occurring on day 4 - 5 from introduction of the 4 factors was suggested.

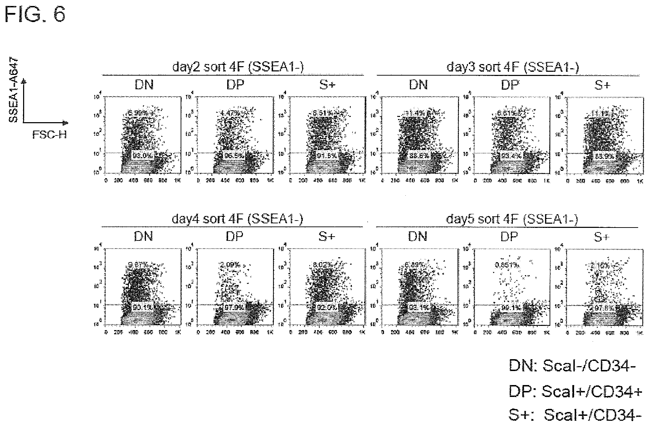

Using expression of SSEA1 as an index of iPS cell formation instead of Nanog, the same experiment as in Fig. 5 was performed (Fig. 6). When sorted on day 2 from the introduction of the 4 factors, the frequency of emergence of iPS cell colonies does not vary among 3 cell populations, but when sorted on day 3, the frequency of emergence of iPS cell colonies in Sca-1⁻/CD34⁻ cell population tended to be high. When sorted on day 4 and day 5, iPS cells mainly emerged from Sca-1⁻/CD34⁻ cell population. The tendency of emergence of iPS cell mainly from Sca-1⁻/CD34⁻ cell population was also maintained on day 5 and thereafter. Thus, a possibility of strong fate determination to be iPS cell occurring on day 4 - 5 from introduction of the 4 factors was suggested.

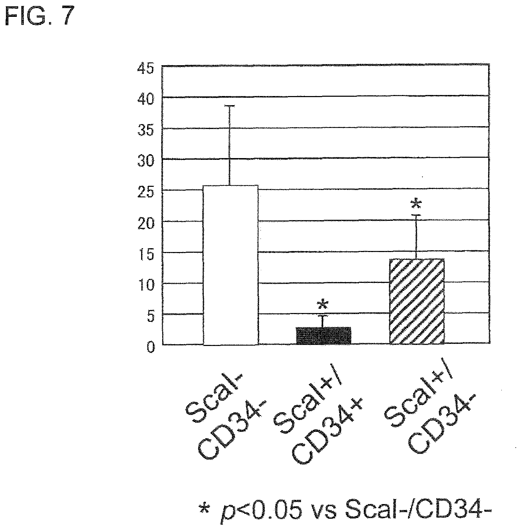

On day 5 from introduction of the 4 factors, each cell population of Sca-1⁻/CD34⁻, Sca-1⁺/CD34⁺, Sca-1⁺/CD34⁻ was sorted by a cell sorter, the cells were replated on feeder cells and cultured again under conditions suitable for iPS cell formation. On day 14 - 21 of culture, the colonies of iPS cells were visualized by an anti-Nanog antibody, and the number of colonies was counted (Fig. 7). Calculating from Sca-1⁻/CD34⁻ population being about 5% of the total cells, Sca-1⁺/CD34⁺ population being about 35% of the total cells, and the iPS cell formation efficiency of the whole being 2 - 3%, it was demonstrated that iPS cells were formed from Sca-1⁻/CD34⁻ population at a very high efficiency of about 30%.

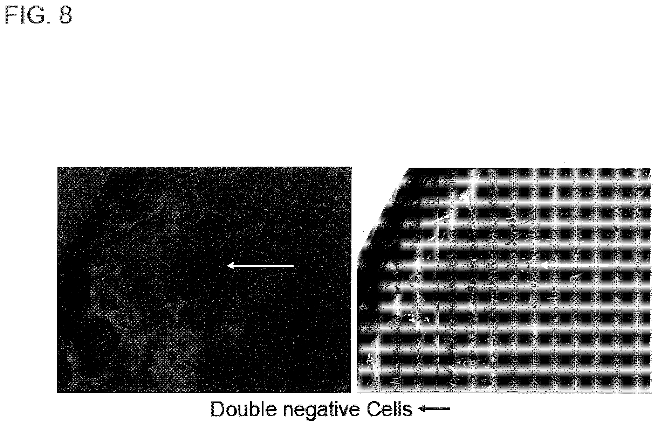

The morphology of each cell population was observed by a fluorescence microscope on day 5 from introduction of the 4 factors (Fig. 8). Sca-1 is shown in green (FITC), and CD34 is shown in red (PE). It was observed that Sca-1⁻/CD34⁻ cells became compact and were closely adhered to each other to form a colony. Sca-1⁻/CD34⁻ cells showed a monolayer epithelial cell-like morphology which is like a intermediate of fibroblast and iPS cell.

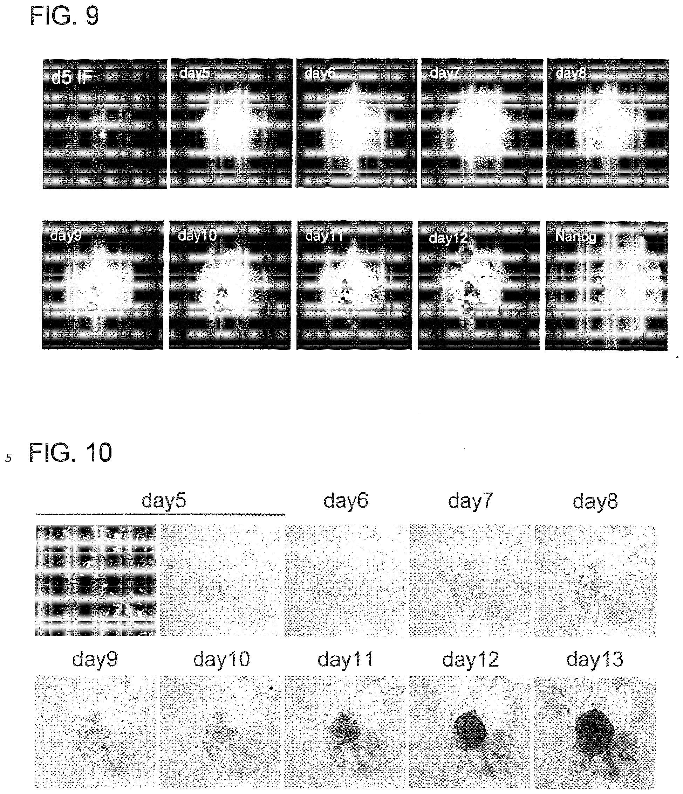

On day 5 from introduction of the 4 factors, cells were stained alive (Sca-1 is green (FITC), CD34 is red (PE)), and culture was continued to induce iPS cells (Figs. 9, 10). Formation of iPS cells from Sca-1⁻/CD34⁻ cell population was observed.

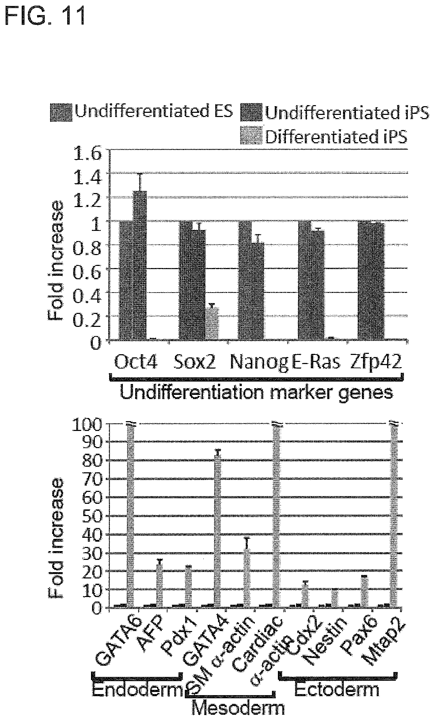

The gene expression in iPS cells established from Sca-1⁻ /CD34⁻ cell population sorted by a cell sorter on day 5 from introduction of the 4 factors was evaluated by quantitative RT-PCR (Fig. 11). As a result, in the same manner as undifferentiated ES cells, each gene of Oct4, Sox2, Nanog, ERas, Zfp42 was expressed and marker genes specific to three differentiated germ layers were not expressed.

Establishment of a chimeric mouse from iPS cells established from Sca-1⁻/CD34⁻ cell population sorted by a cell sorter on day 5 from introduction of the 4 factors was tried. As a result, a chimeric mouse containing a hair color (black) derived from iPS cell was successfully established. The chimeric mouse was crossed with a wild-type mouse, and an offspring mouse with a hair color (black) derived from iPS cell was born. The results have confirmed that iPS cells established from Sca-1⁻/CD34⁻ cell population have an ability to differentiate into all germ layers including germ line (pluripotency).

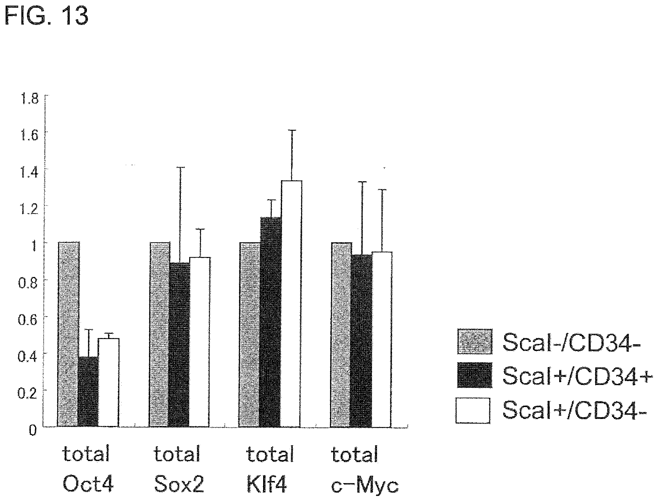

The expression of each reprogramming factor (Oct4, Sox2, Klf4, c-Myc) in each cell population of Sca-1⁻/CD34⁻, Sca-1⁺/CD34⁺, Sca-1⁺/CD34⁻ sorted by a cell sorter on day 5 from introduction of the 4 factors was evaluated by quantitative RT-PCR (Fig. 13). As a result, a mild increase only in the Oct4 expression was found in Sca-1⁻/CD34⁻ cell population, but a significant difference was not found in the expression of other factors.

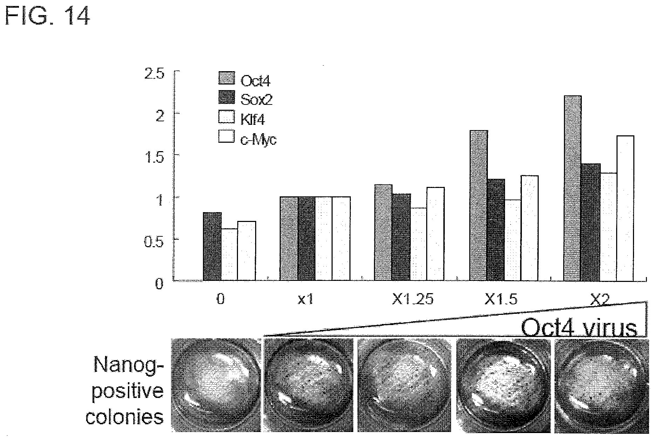

From Fig. 13, high Oct4 expression was considered to have possibly caused high iPS cell formation frequency. Therefore, the Oct4 virus amount was changed, and the iPS cell formation frequency from Sca-1⁻/CD34⁻ cell population was compared (Fig. 14). As a result, the induction efficiency of iPS cell did not differ much even when the Oct4 virus amount was increased to raise the expression amount thereof. Therefore, it was shown that a high level of forced expression of Oct4 alone cannot explain the reason for high (about 30%) iPS cell induction efficiency of Sca-1⁻/CD34⁻ cell population.

### [Example 2] Production of myocardial cells

According to the method described in Takahashi, K. and Yamanaka, S., Cell, 126: 663-676 (2006), 4 factors (Oct3/4, Sox2, Klf4, c-Myc) derived from mouse were introduced into mouse embryonic fibroblasts by a retrovirus, and the fibroblasts were cultured in a medium (FBS mES medium without LIF). The medium composition is as described below.

**Table 2**

| |
|---|
| DMEM (Invitrogen, 11995) 425ml |
| Fetal Bovine Serum (ES grade, heat inactivated) 50ml |
| 100x Non-essential amino acid (Millipore, TMS-001-C) 5ml |
| 100x Nucleosides (Millipore, ES-008-D) 5ml |
| Sodium pyruvate (x100) (Invitrogen, 11360-070) 5ml |
| Penicillin-Streptmycin(x100) (Invitrogen, 15140-122) 5ml |
| Glutamax(x100) (Invitrogen, 35050-061) 5ml |
| 2-Mercaptoethanol (x1000) (Invitrogen, 21985-023) 1ml |

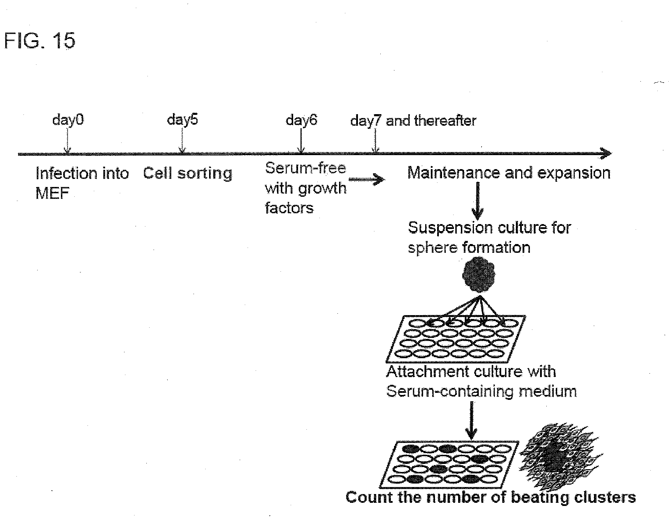

On day 5 from the introduction, each cell population of Sca-1⁻/CD34⁻, Sca-1⁺/CD34⁺, Sca-1⁺/CD34⁻ was sorted by a cell sorter, the cells were cultured for one day, and then differentiation into a cardiac progenitor cell was tried by cultivation in a serum-free medium containing bFGF and EGF according to the method described in Tomita Y et al. J Cell Biol. 2005; 170:1135-46. Furthermore, the obtained cardiac progenitor cells were subjected to floating culture according to the method described in Tomita Y et al. J Cell Biol. 2005; 170:1135-46 to form a sphere. Differentiation into myocardial cell was tried by subjecting the obtained spheres to adhesion culture in a serum containing medium. The cells were replated on feeder cells (Fig. 15).

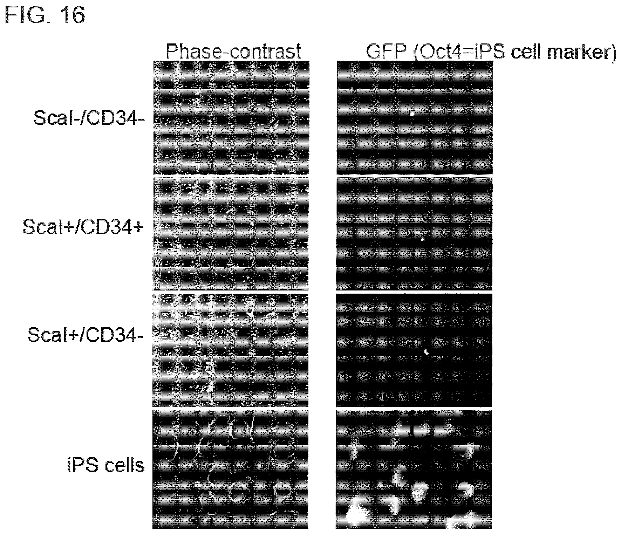

On day 5 from the introduction of the 4 factors, each cell population of Sca-1⁻/CD34⁻, Sca-1⁺/CD34⁺, Sca-1⁺/CD34⁻ was sorted by a cell sorter, and the cells were subjected to adhesion culture in a cardiac progenitor cell induction medium. However, Oct4-GFP positive iPS cell colonies did not emerge. In addition, the morphology of the cell was different from that of iPS cell (Fig. 16).

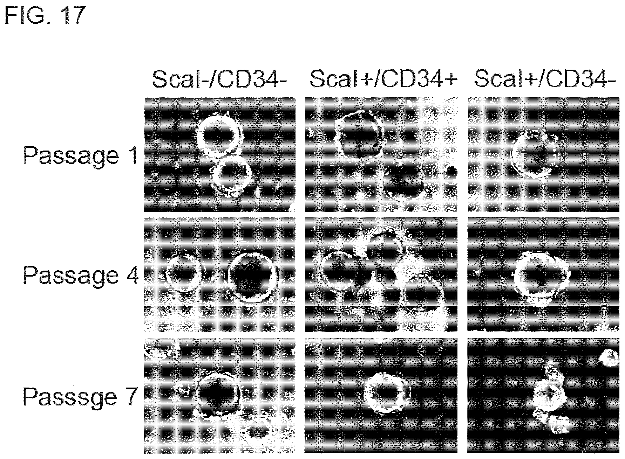

On day 5 from the introduction of the 4 factors, each cell population of Sca-1⁻/CD34⁻, Sca-1⁺/CD34⁺, Sca-1⁺/CD34⁻ was sorted by a cell sorter, and the cells were subjected to adhesion culture in a cardiac progenitor cell induction medium. As a result, the phenotype thereof was maintained up to 10 passages. When the cells after adhesion culture were subjected to floating culture, they showed sphere formation potency, which is one of the characteristics of cardiac progenitor cell (Fig. 17).

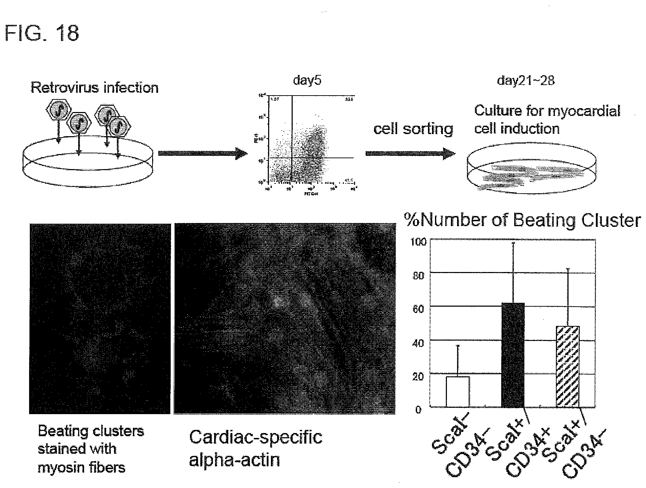

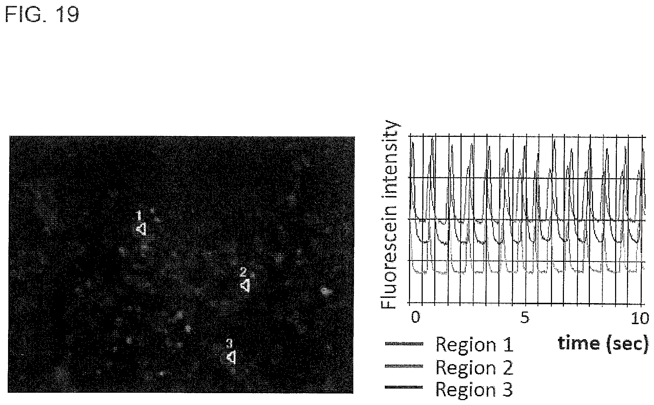

On day 5 from the introduction of the 4 factors, each cell population of Sca-1⁻/CD34⁻, Sca-1⁺/CD34⁺, Sca-1⁺/CD34⁻ was sorted by a cell sorter, and the cells were subjected to adhesion culture (up to 10 passages) in a cardiac progenitor cell induction medium, and then spheres were formed. The obtained spheres were again subjected to adhesion culture to successfully induce self-beating myocardial cell clusters (Fig. 18). The myocardial cell clusters were most efficiently induced from Sca-1⁺/CD34⁺ cell population. It was observed that the intracellular calcium concentration of the induced myocardial cell cluster oscillates in synchronization with self-beating (Fig. 19).

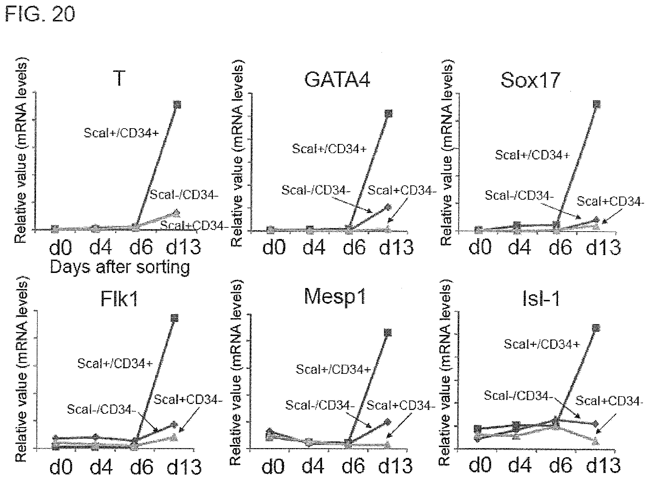

Expression of cardiac progenitor cell marker genes was measured over time (Fig. 20). As a result, it was shown that adhesion culture in a cardiac progenitor cell induction medium results in high expression of each cardiac progenitor cell marker gene in Sca-1⁺/CD34⁺ cell population.

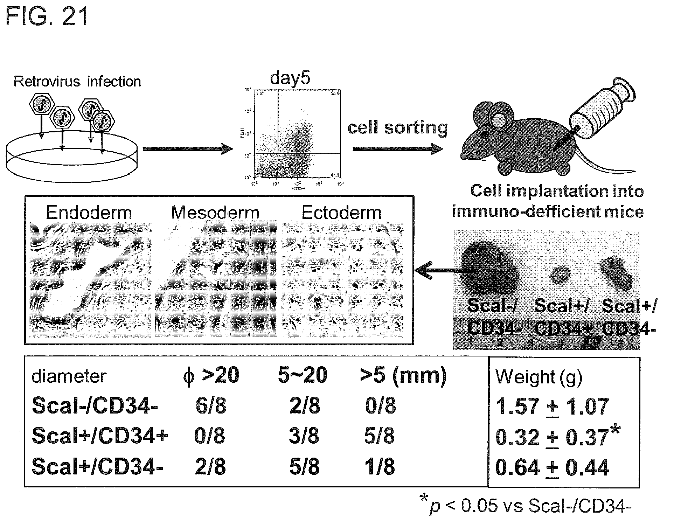

On day 5 from the introduction of the 4 factors, each cell population of Sca-1⁻/CD34⁻, Sca-1⁺/CD34⁺, Sca-1⁺/CD34⁻ was sorted by a cell sorter, each cell population was transplanted into immunodeficient mouse (NOD/Shi-scid IL2rgamma (null) (NOG) mice) (Ito M et al. Curr Top Microbiol Immunol. 2008; 324:53-76), and teratoma formation capacity was evaluated (Fig. 21). As a result, Sca-1⁺/CD34⁺ cell population did not form a teratoma exceeding diameter 20 mm. Therefore, it was shown that Sca-1⁺/CD34⁺ cell population is useful as a source of myocardial cell having a low risk of tumor formation.

### [Example 3] Production of induced pluripotent stem cells by 3 factors

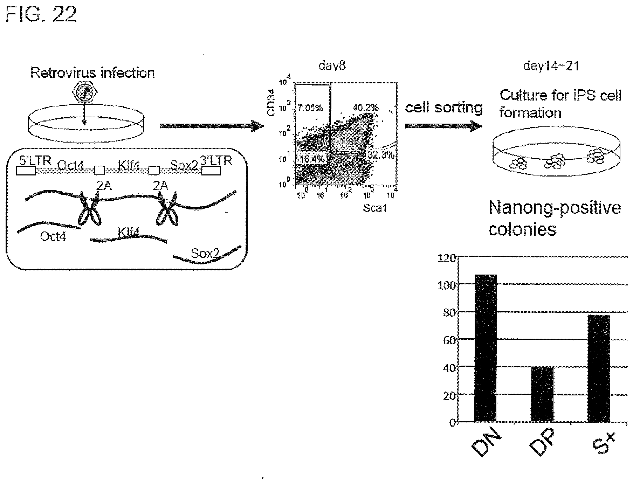

According to the method described in Nakagawa, M. et al., Nat. Biotethnol., 26: 101-106 (2008), 3 factors (Oct3/4, Sox2, Klf4) derived from mouse were introduced into mouse embryonic fibroblasts by a retrovirus, and the fibroblasts were cultured in a medium (FBS mES medium without LIF). After 8 days from introduction of the 3 factors, each cell population of Sca-1⁻ /CD34⁻, Sca-1⁺/CD34⁺, Sca-1⁺/CD34⁻ was isolated by a cell sorter, and the cells were replated on feeder cells and cultured again under conditions suitable for iPS cell formation. As the feeder cell, MEFs treated with mitomycin C to stop cell division were used. On day 14 - 21 of culture, the colonies of iPS cells were visualized by an anti-Nanog antibody, and the frequency of emergence of iPS cell colonies was compared (Fig. 22). As a result, iPS cell colonies were shown to emerge at a high efficiency from the cell population of Sca-1⁻/CD34⁻ even when 3 factors were introduced.

### [Example 4] Production of induced pluripotent stem cells

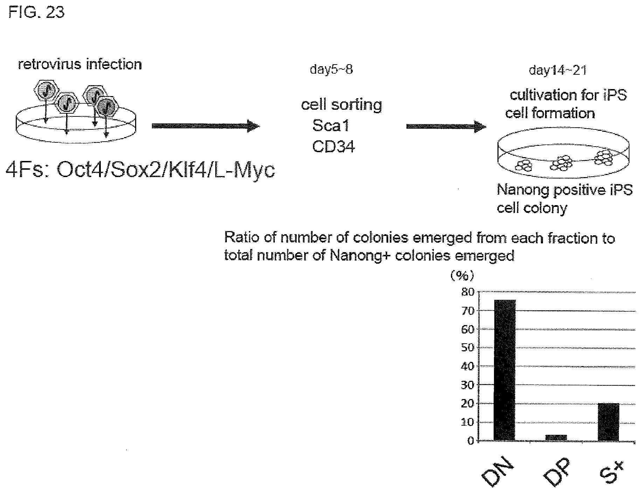

Under the conditions same as those of Example 1 except that L-Myc was used instead of c-Myc, pluripotent stem cells were established from mouse embryonic fibroblasts. On day 5 - 8 from introduction of the 4 factors (Oct3/4, Sox2, Klf4, L-Myc), each cell population of Sca-1⁻/CD34⁻, Sca-1⁺/CD34⁺, Sca-1⁺/CD34⁻ was sorted by a cell sorter, the cells were replated on feeder cells and cultured again under conditions suitable for iPS cell formation. On day 14 - 21 of culture, the number of colonies of Nanog positive iPS cells was counted (Fig. 23). As a result, iPS cells mainly emerged from Sca-1⁻/CD34⁻ cell population, and iPS cell colony emerged from cell population Sca-1⁺/CD34⁺ was few. Therefore, a possibility of strong fate determination to be iPS cell occurring on day 4 - 5 from introduction of the 4 factors was suggested. Therefore, irrespective of the kind of combination of reprogramming factors, a possibility of induction of iPS cells at a high frequency from Sca-1⁻/CD34⁻ cell population was suggested.

### [Example 5] Search for alternative marker of Sca-1

The 4 factors (Oct3/4, Sox2, Klf4, c-Myc) were introduced into mouse embryonic fibroblasts, a cellular surface marker gene showing high expression in Sca-1⁻/CD34⁻ cell population sorted by a cell sorter on day 5 from the introduction was comprehensively searched for by microarray, and EpCam was identified. Whether EpCam functions as a reprogramming success marker replacing Sca-1 was tested using human iPS cell.

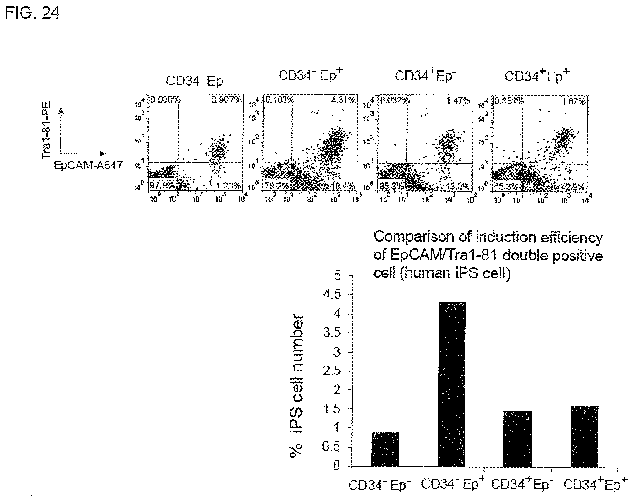

Reprogramming factors (Oct3/4, Sox2, Klf4, c-Myc) were introduced into newborn human fibroblasts by a retrovirus, each cell population of EpCam⁻/CD34⁻, EpCam⁺/CD34⁻, EpCam⁻/CD34⁺, EpCam⁺/CD34⁺ was sorted on day 7 from the introduction, the cells were replated on feeder cells and further cultured under conditions suitable for iPS cell formation to induce iPS cell. The number of induced iPS cells was counted by analyzing the expression of Tra1-81, which is a marker of undifferentiated human iPS cell, by flow cytometer. As a result, iPS cells were induced at the highest frequency from the cell population of EpCam⁺/CD34⁻ (Fig. 24). The results show that EpCam functions as a reprogramming success marker, similar to Sca-1.

### Industrial Applicability

According to the present invention, since a surface marker important for the prediction of a reprogramming success of a somatic cell has been identified, a cell population having high possibility of success in reprogramming can be easily isolated in an early stage after introduction of reprogramming factors. Moreover, since reprogramming success candidate cells can be narrowed down, more accurate and detailed elucidation of the molecular mechanism relating to the reprogramming is possible. Furthermore, the present invention provides a source of myocardial cell having a low risk of teratoma formation. Therefore, the present invention is useful in the field of regenerative medicine.

This application is based on a patent application No. 2012-288955 filed in Japan (filing date: December 28, 2012), the contents of which are incorporated in full herein.

## Claims

1. A method for the production of an induced pluripotent stem cell comprising the following steps:
(1) contacting nuclear reprogramming substances with somatic cells,
(2) culturing the cells obtained in (1),
(3) isolating a Sca-1-negative CD34-negative cell or EpCam-positive CD34-negative cell from the cells obtained in (2), and
(4) further culturing the cell isolated in (3) to give an induced pluripotent stem cell.

2. The method according to claim 1, wherein the nuclear reprogramming substances are Oct3/4, Klf4 and Sox2, or nucleic acids encoding the same.

3. The method according to claim 1 or 2, wherein the nuclear reprogramming substances are Oct3/4, Klf4, Sox2 and c-Myc or L-Myc, or nucleic acids encoding the same.

4. The method according to any one of claims 1 to 3, wherein the somatic cells are rodent somatic cells or human somatic cells.

5. The method according to any one of claims 1 to 4, wherein the somatic cells are fibroblasts.

6. A method of concentrating a cell capable of forming an induced pluripotent stem cell from somatic cells contacted with nuclear reprogramming substances, comprising isolating a Sca-1-negative CD34-negative cell or EpCam-positive CD34-negative cell from somatic cells contacted with nuclear reprogramming substances.

7. Use of a reagent comprising an antibody specifically recognizing Sca-1 or EpCam and an antibody specifically recognizing CD34, for concentrating a cell capable of forming an induced pluripotent stem cell from somatic cells contacted with nuclear reprogramming substances.

8. A method for the production of a myocardial cell or a progenitor cell thereof, comprising the following steps:
(1') contacting nuclear reprogramming substances with somatic cells,
(2') culturing the cells obtained in (1'),
(3') isolating a Sca-1-positive CD34-positive cell or EpCam-negative CD34-positive cell from the cells obtained in (2'), and
(4') culturing the cell isolated in (3') under a myocardial cell or its progenitor cell differentiation condition to give a myocardial cell or a progenitor cell thereof.

9. The method according to claim 8, wherein the nuclear reprogramming substances are Oct3/4, Klf4 and Sox2, or nucleic acids encoding the same.

10. The method according to claim 8 or 9, wherein the nuclear reprogramming substances are Oct3/4, Klf4, Sox2 and c-Myc or L-Myc, or nucleic acids encoding the same.

11. The method according to any one of claims 8 to 10, wherein the somatic cells are rodent somatic cells or human somatic cells.

12. The method according to any one of claims 8 to 11, wherein the somatic cells are fibroblasts.

13. A method of concentrating a cell which has a low possibility of forming induced pluripotent stem cells but has a high differentiation potency into myocardial cell or a progenitor cell thereof, from somatic cells contacted with nuclear reprogramming substances, comprising isolating a Sca-1-positive CD34-positive cell or EpCam-negative CD34-positive cell from somatic cells contacted with nuclear reprogramming substances.

14. Use of a reagent comprising an antibody specifically recognizing Sca-1 or EpCam and an antibody specifically recognizing CD34, for concentrating a cell which has a low possibility of forming induced pluripotent stem cells but has a high differentiation potency into myocardial cell or a progenitor cell thereof from somatic cells contacted with nuclear reprogramming substances.

## Patentansprüche

1. Verfahren zur Herstellung einer induzierten, pluripotenten Stammzelle, umfassend die folgenden Schritte:
(1) In-Kontakt-Bringen nukleärer Reprogrammierungssubstanzen mit somatischen Zellen,
(2) Kultivieren der Zellen, die in (1) erhalten wurden,
(3) Isolieren einer Sca-1-negativen, CD34-negativen Zelle oder EpCam-positiven, CD34-negativen Zelle aus den Zellen, die in (2) erhalten wurden, und
(4) weiteres Kultivieren der Zelle, die in (3) isoliert wurde, um eine induzierte, pluripotente Stammzelle ergeben.

2. Verfahren nach Anspruch 1, wobei die nukleären Reprogrammierungssubstanzen Oct3/4, Klf4 und Sox2 sind oder Nukleinsäuren, die selbige kodieren.

3. Verfahren nach Anspruch 1 oder 2, wobei die nukleären Reprogrammierungssubstanzen Oct3/4, Klf4, Sox2 und c-Myc oder L-Myc sind oder Nukleinsäuren, die selbige kodieren.

4. Verfahren nach irgendeinem der Ansprüche 1 bis 3, wobei die somatischen Zellen somatische Zellen vom Nager oder humane, somatische Zellen sind.

5. Verfahren nach irgendeinem der Ansprüche 1 bis 4, wobei die somatischen Zellen Fibroblasten sind.

6. Verfahren zur Konzentrierung einer Zelle, die in der Lage ist, aus somatischen Zellen, die mit nukleären Reprogrammierungssubstanzen in Kontakt gebracht sind, eine induzierte, pluripotente Stammzelle zu bilden, umfassend das Isolieren einer Sca-1-negativen, CD34-negativen Zelle oder EpCam-positiven, CD34-negativen Zelle aus somatischen Zellen, die mit nukleären Reprogrammierungssubstanzen in Kontakt gebracht sind.

7. Verwendung eines Reagenzes, umfassend einen Antikörper, der spezifisch Sca-1 oder EpCam erkennt und einen Antikörper, der spezifisch CD34 erkennt, zur Konzentrierung einer Zelle, die in der Lage ist, aus somatischen Zellen, die mit nukleären Reprogrammierungssubstanzen in Kontakt gebracht wurden, eine induzierte, pluripotente Stammzelle zu bilden.

8. Verfahren zur Herstellung einer myokardialen Zelle oder einer Vorläuferzelle davon, umfassend die folgenden Schritte:
(1') In-Kontakt-Bringen nukleärer Reprogrammierungssubstanzen mit somatischen Zellen,
(2') Kultivieren der Zellen, die in (1') erhalten wurden,
(3') Isolieren einer Sca-1-positiven, CD34-positiven Zelle oder EpCam-negativen, CD34-positiven Zelle aus den Zellen, die in (2') erhalten wurden, und
(4') Kultivieren der Zelle, die in (3') isoliert wurde, unter Differenzierungsbedingung einer myokardialen Zelle oder deren Vorläuferzelle, um eine myokardiale Zelle oder deren Vorläuferzelle zu ergeben.

9. Verfahren nach Anspruch 8, wobei die nukleären Reprogrammierungssubstanzen Oct3/4, Klf4 und Sox2 sind oder Nukleinsäuren, die selbige kodieren.

10. Verfahren nach Anspruch 8 oder 9, wobei die nukleären Reprogrammierungssubstanzen Oct3/4, Klf4, Sox2 und c-Myc oder L-Myc sind oder Nukleinsäuren, die selbige kodieren.

11. Verfahren nach irgendeinem der Ansprüche 8 bis 10, wobei die somatischen Zellen somatische Zellen vom Nager oder humane, somatische Zellen sind.

12. Verfahren nach irgendeinem der Ansprüche 8 bis 11, wobei die somatischen Zellen Fibroblasten sind.

13. Verfahren zum Konzentrieren einer Zelle, die aus somatischen Zellen, die mit nukleären Reprogrammierungssubstanzen in Kontakt gebracht sind, ein geringes Vermögen aufweist, induzierte, pluripotente Stammzellen zu bilden, aber ein hohes Differenzierungspotenzial in eine myokardiale Zelle oder eine Vorläuferzelle davon aufweist, umfassend das Isolieren einer Sca-1-positiven, CD34-positiven Zelle oder EpCam-negativen, CD34-positiven Zelle aus somatischen Zellen, die mit nukleären Reprogrammierungssubstanzen in Kontakt gebracht sind.

14. Verwendung eines Reagenzes, umfassend einen Antikörper, der spezifisch Sca-1 oder EpCam erkennt und einen Antikörper, der spezifisch CD34 erkennt, zur Konzentrierung einer Zelle, die aus somatischen Zellen, die mit nukleären Reprogrammierungssubstanzen in Kontakt gebracht sind, ein geringes Vermögen aufweist, induzierte, pluripotente Stammzellen zu bilden, aber einen hohes Differenzierungspotenzial in eine myokardiale Zelle oder eine Vorläuferzelle davon aufweist.

## Revendications

1. Méthode pour la production d'une cellule souche pluripotente induite comprenant les étapes suivantes :
(1) la mise en contact de substances de reprogrammation nucléaire avec des cellules somatiques,
(2) la culture des cellules obtenues en (1),
(3) l'isolement d'une cellule Sca-1-négative CD34-négative ou d'une cellule EpCam-positive CD34-négative à partir des cellules obtenues en (2), et
(4) la culture supplémentaire de la cellule isolée en (3) pour donner une cellule souche pluripotente induite.

2. Méthode selon la revendication 1, dans laquelle les substances de reprogrammation nucléaire sont Oct3/4, Klf4 et Sox2, ou des acides nucléiques codant celles-ci.

3. Méthode selon la revendication 1 ou 2, dans laquelle les substances de reprogrammation nucléaire sont Oct3/4, Klf4, Sox2 et c-Myc ou L-Myc, ou des acides nucléiques codant celles-ci.

4. Méthode selon l'une quelconque des revendications 1 à 3, dans laquelle les cellules somatiques sont des cellules somatiques de rongeur ou des cellules somatiques humaines.

5. Méthode selon l'une quelconque des revendications 1 à 4, dans laquelle les cellules somatiques sont des fibroblastes.

6. Méthode de concentration d'une cellule capable de former une cellule souche pluripotente induite à partir de cellules somatiques mises en contact avec des substances de reprogrammation nucléaire, comprenant l'isolement d'une cellule Sca-1-négative CD34-négative ou d'une cellule EpCam-positive CD34-négative à partir des cellules somatiques mises en contact avec des substances de reprogrammation nucléaire.

7. Utilisation d'un réactif comprenant un anticorps reconnaissant spécifiquement Sca-1 ou EpCam et un anticorps reconnaissant spécifiquement CD34, pour la concentration d'une cellule capable de former une cellule souche pluripotente induite à partir de cellules somatiques mises en contact avec des substances de reprogrammation nucléaire.

8. Méthode pour la production d'une cellule myocardique ou d'une cellule progéniteur de celle-ci, comprenant les étapes suivantes :
(1') la mise en contact de substances de reprogrammation nucléaire avec des cellules somatiques,
(2') la culture des cellules obtenues en (1'),
(3') l'isolement d'une cellule Sca-1-positive CD34-positive ou d'une cellule EpCam-négative CD34-positive à partir des cellules obtenues en (2'), et
(4') la culture de la cellule isolée en (3') dans des conditions de différenciation de la cellule myocardique ou de sa cellule progéniteur pour donner une cellule myocardique ou une cellule progéniteur de celle-ci.

9. Méthode selon la revendication 8, dans laquelle les substances de reprogrammation nucléaire sont Oct3/4, Klf4 et Sox2, ou des acides nucléiques codant celles-ci.

10. Méthode selon la revendication 8 ou 9, dans laquelle les substances de reprogrammation nucléaire sont Oct3/4, Klf4, Sox2 et c-Myc ou L-Myc, ou des acides nucléiques codant celles-ci.

11. Méthode selon l'une quelconque des revendications 8 à 10, dans laquelle les cellules somatiques sont des cellules somatiques de rongeur ou des cellules somatiques humaines.

12. Méthode selon l'une quelconque des revendications 8 à 11, dans lequel les cellules somatiques sont des fibroblastes.

13. Méthode de concentration d'une cellule qui présente une faible possibilité de former des cellules souches pluripotentes induites mais présente une puissance de différenciation élevée en une cellule myocardique ou une cellule progéniteur de celle-ci, à partir de cellules somatiques mises en contact avec des substances de reprogrammation nucléaire, comprenant l'isolement d'une cellule Sca-1-positive CD34-positive ou d'une cellule EpCam-négative CD34-positive à partir de cellules somatiques mises en contact avec des substances de reprogrammation nucléaire.

14. Utilisation d'un réactif comprenant un anticorps reconnaissant spécifiquement Sca-1 ou EpCam et un anticorps reconnaissant spécifiquement CD34, pour la concentration d'une cellule qui présente une faible possibilité de former des cellules souches pluripotentes induites mais présente une puissance de différenciation élevée en une cellule myocardique ou une cellule progéniteur de celle-ci à partir de cellules somatiques mises en contact avec des substances de reprogrammation nucléaire.
